# EUROPÄISCHE PATENTANMELDUNG

(11) **EP 2 911 054 A1**
(43) Veröffentlichungstag der Anmeldung: **26.08.2015**
(21) Anmeldenummer: 14156001.1
(22) Anmeldetag: 20.02.2014
(51) Int. Cl.: G06F 9/44

(54) **System zur Verwaltung und Bearbeitung von Daten einer medizinischen Einrichtung**

(71) Anmelder: Siemens Aktiengesellschaft, 80333 München (DE)
(72) Erfinder: Dorn, Karlheinz, 90562 Kalchreuth (DE)

(57) **Zusammenfassung**

Es wird ein System (1) zur Verwaltung und Bearbeitung von Daten einer medizinischen Einrichtung (2, 2') angegeben. Das System (1) umfasst mindestens eine Browser-Applikation (31-33) zum Ablauf auf einem Nutzergerät (8), die dazu eingerichtet ist, Datensatzlisten (35), die zu einer Anzahl von medizinischen Datensätzen (P,W,C) mindestens einer bestimmten Datenart jeweils einen Listeneintrag (27) enthalten, sowie einzelne Listeneinträge (27) zur Ansicht und Bearbeitung anzuzeigen. Die Browser-Applikation (31-33) hat eine mehrschichtige Komponenten-Architektur mit einer Rahmenschicht (90), einer View-Schicht (92), einer View-Model-Schicht (95), einer Model-Schicht (98) und einer Treiberschicht (100). Die Komponenten (96,97) der ViewModel-Schicht (95) sind hierbei generisch, das heißt unabhängig von der Datenart.

## Beschreibung

Die Erfindung bezieht sich auf ein System zur Verwaltung und Bearbeitung von Daten einer medizinischen Einrichtung.

Unter dem Begriff "Verwaltung" wird hier und im Folgenden insbesondere die Archivierung der Daten (das heißt die Einstellung der Daten in einen persistenten Datenspeicher), die Wiedergabe (Anzeige) und das Löschen der Daten aus dem Datenspeicher sowie das Sortieren und Auffinden bestimmter Daten aus dem Datenspeicher nach Maßgabe vorgegebener Kriterien (Browsing) verstanden.

Unter dem Begriff "Bearbeitung" wird hier und im Folgenden insbesondere die Änderung (Editierung / Aufbereitung) der Daten verstanden.

Die zu verwaltenden und zu bearbeitenden Daten der medizinischen Einrichtung umfassen insbesondere Patientendaten, Aufgaben (Works/Tasks) oder Aufgabenlisten (Worklists) für das Personal der medizinischen Einrichtung und medizinische Bilddaten.

Die Verwaltung solcher medizinischer Daten erfolgt in zunehmendem Maße computergestützt durch Server-Systeme, insbesondere sogenannte Informationssysteme. Ein Informationssystem umfasst regelmäßig
- einen oder mehrere Datenspeicher, beispielsweise in Form eines Storage Area Network (SAN),
- einen oder mehrere zugehörige Datenserver,
- mindestens eine relationale Datenbank, die in einem Datenbankserver implementiert ist,
- sowie in der Regel einen oder mehrere weitere Server, in denen Methoden für den Datenbankzugriff und die Datenbearbeitung implementiert sind.

Für die unterschiedlichen Datenarten haben sich im medizinischen Bereich unterschiedliche Informationssysteme etabliert. So werden im Umfeld einer medizinischen Einrichtung wie zum Beispiel einer Klinik regelmäßig ein "Krankenhaus-Informations-System" (Hospital Information System, HIS) für die Verwaltung und Bearbeitung der Patientendaten und ein "Radiologie-Informations-System" (RIS) für die Terminplanung radiologischer Untersuchungen, die Unterstützung der Befundung medizinischer Bilddaten und die Dokumentation der Befunde eingesetzt. Daneben umfasst die IT-Struktur eines Krankenhauses in der Regel ein sogenanntes "Picture Archiving and Communication System" (PACS) zur Archivierung und Übertragung von medizinischen Bilddaten auf Basis des DICOM-Standards sowie ein "Avanced Visualisation(AV)-System", das servergestützte Funktionen zur Visualisierung von Volumendaten, insbesondere dynamisches Volume Rendering, zur Verfügung stellt.

Die vorstehend bezeichneten Server-Systeme sind dabei in der Regel parallel zueinander vorhanden. Dies erfordert einen hohen Beschaffungs- und Wartungsaufwand, der insbesondere für kleine medizinische Einrichtungen oder sonstige Einrichtung mit vergleichsweise geringem Finanzvolumen kaum zu bewältigen ist.

Die vorstehend beschriebene, komplexe IT-Struktur einer modernen medizinischen Einrichtung weist zudem nur vergleichsweise schlechte Skalierungseigenschaften auf. Eine Anpassung einer solchen IT-Struktur an größere Änderungen des zu verarbeitenden und zu archivierenden Datenvolumens und/oder der erforderlichen Rechenleistung ist somit meist nur mit sehr hohem Aufwand möglich.

Als Nutzer- oder Endgeräte (klassischerweise als Clients bezeichnet) einer solchen IT-Struktur werden bisher überwiegend Personal Computer (PCs) verwendet, wobei diese PCs oft als sogenannte "Thin Clients" ausgebildet sind, die einen Großteil der erforderlichen Rechenleistung von einem angeschlossenen Server beziehen. In jüngerer Zeit wird allerdings zunehmend gewünscht, auch mobile Kleinrechner wie Smartphones, Tablets oder PDAs als Nutzergerät zu verwenden.

Ein weiteres Problem herkömmlicher Informations-Systeme im medizinischen Umfeld besteht darin, dass die Frontend-Software dieser Systeme oft spezifisch und starr auf die Verwaltung und Bearbeitung bestimmter Datenarten ausgerichtet ist. Dies führt dazu, dass das Frontend für jedes Informationssystem eigens programmiert und gepflegt werden muss. Dies wiederum erschwert insbesondere die Integration neuartiger Nutzergeräte wie Smartphones und Tablets in den klinischen Arbeitsablauf, da die mit der entsprechenden Anpassung der jeweiligen Frontends verbundene Diversifizierung der Softwarekomponenten vom Herstellungs- und Weiterentwicklungsaufwand her nur mit großem Aufwand handhabbar ist.

Als Alternative zu herkömmlichen Client-Server-Architekturen haben sich in den vergangenen Jahren zunehmend sogenannte Cloud-Lösungen etabliert. Als "Cloud" ("Rechnerwolke") wird dabei eine Datenverarbeitungseinrichtung verstanden, die von einem von dem Nutzer unabhängigen Cloud-Betreiber ("Cloud Vendor") zur Verfügung gestellt und betrieben wird. Der "Cloud Vendor" stellt hierbei einer Vielzahl von Nutzern die Hardware und gegebenenfalls die Software der Cloud im Rahmen eines Nutzungsvertrags (Subscription) als Dienst zur Verfügung. Je nach dem Umfang der zur Verfügung gestellten Dienste unterscheidet man zwischen
- einem als "Infrastructure as a Service" (IaaS) bezeichneten Nutzungsmuster, bei dem dem Nutzer lediglich Computerhardware (Rechner, Netzwerke und Speicher) der Cloud zur Verfügung gestellt wird, während der Nutzer für die in der Cloud betriebene Software in vollem Umfang selbst verantwortlich ist,
- einem als "Platform as a Service" (PaaS) beschriebenen Nutzungsmuster, bei dem dem Nutzer aus der Cloud die Computerhardware zusammen mit einer darauf aufbauenden Programmierungs- und Laufzeitumgebung angeboten wird, so dass der Nutzer nur für die in dieser Programmierungs- und Laufzeitumgebung implementierte Anwendungssoftware (Applikationen) selbst verantwortlich ist, sowie
- einem als "Software as a Service" (SaaS) bezeichneten Nutzungsmuster, bei dem dem Nutzer darüber hinaus auch bestimmte Anwendungssoftware aus der Cloud zur Verfügung gestellt wird.
Je nach dem Nutzerkreis, an den die jeweilige Cloud adressiert ist, unterscheidet man des Weiteren zwischen
- einer sogenannten Public Cloud, deren Dienste von jedem in Anspruch genommen werden können, und
- einer sogenannten Privat Cloud, die nur Nutzern einer bestimmten Organisation, insbesondere eines bestimmten Konzerns zugänglich ist.

Für jeden Nutzer einer Public Cloud sind die Zugriffsberechtigungen auf bestimmte Hardware- und Softwarebestandteile der Cloud durch die dem Nutzer zugeordnete Subscription geregelt. Public Clouds sind hierdurch regelmäßig "mandantenfähig" (multi-tenant). Dies bezeichnet die Fähigkeit, Daten, Benutzerverwaltung und Rechenoperationen für Nutzer mit verschiedener Subscription strikt getrennt zu halten. Ein Nutzer der Public Cloud kann also in die Daten, Benutzerverwaltung und Rechenoperationen eines anderen Nutzers mit unterschiedlicher Subscription keinen Einblick nehmen und diese Daten auch nicht beeinflussen.

Der Erfindung liegt die Aufgabe zugrunde, ein System zur Verwaltung und Bearbeitung von Daten einer medizinischen Einrichtung anzugeben, das besonders flexibel einsetzbar, insbesondere besonders skalierbar ist.

Diese Aufgabe wird erfindungsgemäß gelöst durch die Merkmale des Anspruchs 1. Vorteilhafte und teils für sich gesehen erfinderische Ausgestaltungen und Weiterentwicklungen der Erfindung sind in den Unteransprüchen und der nachfolgenden Beschreibung dargelegt.

Das erfindungsgemäße System dient insbesondere zur Anzeige und Bearbeitung von medizinischen Datensätzen einer der folgenden Datenarten:
- Patientendaten, die persönliche und medizinische Angaben zu einem bestimmten Patienten der Einrichtung umfassen, insbesondere Angaben zum Namen, der Adresse, dem Alter, dem Geschlecht, früheren Diagnosen und Behandlungen,
- Aufgaben (Works/Tasks) und Aufgabenlisten (Worklists), die von einem oder mehreren medizinischen Nutzern (Ärzten, medizinischen Assistenzkräften, Pflegern etc.) der medizinischen Einrichtung durchzuführen sind, sowie
- Kontextdaten, das heißt Kontextinformation zu medizinischen Bilddaten, insbesondere Verweise auf den Speicherort von Bilddatensätzen sowie optional Meta-Daten zu Bilddatensätzen wie z.B. die der jeweiligen Bildaufnahme zugrundeliegenden Aufnahmeparameter.

Die Datensätze einer bestimmten Datenart sind nachfolgend auch als "Ressource" bezeichnet.

Das System umfasst mindestens eine Browser-Applikation zum Ablauf auf einem Nutzergerät. Die oder jede Browser-Applikation ist hierbei einerseits zur Anzeige von Datensatzlisten eingerichtet, die zu einer Anzahl von medizinischen Datensätzen jeweils einen Listeneintrag enthalten. Diese - nachfolgend als "Feed" bezeichneten - Datensatzlisten sind insbesondere (aber nicht zwangsweise) das Ergebnis einer Suche nach Datensätzen mit bestimmten Eigenschaften, z.B. nach Patientendatensätzen zu Patienten mit einem bestimmten Nachnamen.

Die oder jede Browser-Applikation ist des Weiteren dazu eingerichtet, den Inhalt einzelner Listeneinträge (nachfolgend als "Tweet" bezeichnet) zur Ansicht oder Bearbeitung anzuzeigen. Jeder Tweet kann ganz oder teilweise den Inhalt des zugehörigen Datensatzes selbst enthalten, im Falle von Patientendaten z.B. den Nachnamen, Vornamen, Adresse, etc. eines Patienten, so dass der Datensatz mit dem zugehörigen Tweet ganz oder teilweise identisch sein kann. Vor allem bei Datensätzen mit großem Speicherbedarf sind der Datensatz oder einzelne Komponenten desselben aber bevorzugt getrennt von dem zugehörigen Tweet gespeichert. In diesem Fall umfasst der einem Datensatz zugeordnete Tweet statt der Information des Datensatzes einen oder mehrere Links (insbesondere in Form einer URL), über den auf den Datensatz oder einzelne Komponenten desselben zugegriffen werden kann. Des Weiteren umfasst der einem Datensatz zugehörige Tweet oft auch Links (wiederum vorzugsweise in Form einer URL), die auf den Speicherort eines zugehörigen anderen Datensatzes verweisen. Beispielsweise enthält der einen Patientendatensatz eines Patienten zugeordnete Tweet regelmäßig Links auf Aufgaben und/oder Kontextdatensätze, die ebenfalls mit diesem Patienten verknüpft sind.

Die oder jede Browser-Applikation dient vorzugsweise zur selektiven und ausschließlichen Anzeige und Bearbeitung von Datensätzen einer spezifischen Datenart, so dass beispielsweise zur Verwaltung und Bearbeitung von Patientendaten einerseits sowie zur Anzeige und Bearbeitung von Aufgabenlisten andererseits verschiedene Browser-Applikationen vorgesehen sind. Im Rahmen der Erfindung liegen allerdings auch Ausführungsformen der Browser-Applikation, die zur Verwaltung und Bearbeitung von Datensätzen mehrerer Datenarten vorgesehen sind.

Erfindungsgemäß ist die Browser-Applikation hinsichtlich ihrer Komponenten-Architektur in fünf Schichten gegliedert. Diese Schichten umfassen - entsprechend ihrer Rangordnung von "oben" nach "unten" (d.h. von der Präsentations- und Nutzerinteraktionsebene zu der Datenzugriffsebene) - eine Rahmenschicht, eine View-Schicht, eine ViewModel-Schicht, eine Model-Schicht und eine Treiberschicht.

Die Rahmenschicht umfasst vorzugsweise genau eine Komponente. Die Komponente der Rahmenschicht (nachfolgend "Rahmenkomponente") stellt in zweckmäßiger Ausführung einen generischen Ablauf-Rahmen (Runtime-Container) für die durch die Komponenten der View-Schicht definierten GUI-Seiten dar. Nach außen hin präsentiert sich die "Rahmenkomponente" dabei entweder als native Applikation, die selbstständig auf dem Betriebssystem des Nutzergeräts lauffähig ist, z.B. als IOS-App, Phone-Gap-Exe oder Windows8-App. Sie kann alternativ aber auch zum Ablauf in einem Web-Browser eingerichtet, und in dieser Variante beispielsweise als HTML5-Programm gestaltet sein. Die Rahmenkomponente dient somit vorzugsweise als softwaretechnischer Adapter zwischen der oder jeder GUI-Seite und der Laufzeitumgebung der Applikation, insbesondere dem Betriebssystem des Nutzergeräts oder einem Web-Browser.

Die Rahmenkomponente definiert eine Anzahl und Anordnung von gleichzeitig anzuzeigenden (GUI-)Seiten einer graphischen Nutzeroberfläche (GUI), wobei jede dieser GUI-Seiten entweder zur Anzeige jeweils eines Feeds (also einer Liste von Tweets) oder zur Anzeige des Inhalts eines Tweets dient. Als GUI-Seite wird hierbei ein abgeschlossener und für sich gesehen funktionsfähiger Teil einer graphischen Nutzeroberfläche bezeichnet, der - etwa nach Art eines Fensters - wahlweise alleine oder im Verbund mit weiteren GUI-Seiten betrieben werden kann. Die oder jede Rahmenkomponente umfasst weiterhin Funktionen für die Kontrollübergabe zwischen verschiedenen GUI-Seiten. Als Kontrollübergabe wird hierbei eine - manuell durch einen Nutzer oder automatisch durch das System veranlasste - Aktion bezeichnet, mittels der eine GUI-Seite aktiviert (für eine Nutzerinteraktion freigegeben) oder deaktiviert (für eine Nutzerinteraktion gesperrt), auf dem Bildschirm eingeblendet oder aus dem Bildschirm ausgeblendet wird.

Die oder jede Rahmenkomponente kann hierbei aus Nutzersicht entweder unsichtbar sein oder selbst einen übergeordneten Teil der graphischen Nutzeroberfläche der Browser-Applikation bilden, und dabei insbesondere die oder jede angezeigte GUI-Seite - im Wortsinne - umrahmen.

Dadurch, dass die Rahmenkomponente die Anzahl und Anordnung der angezeigten GUI-Seiten, die Art der Kontrollübergabe sowie gegebenenfalls einen Teil der graphischen Nutzeroberfläche der Browser-Applikation bestimmt, definiert sie - abhängig von der Art des Nutzergeräts - einen an dieses Nutzergerät angepassten Applikationsstil. Beispielsweise werden gemäß einer zur Ausführung der Browser-Applikation auf einem Tablet-Computer vorgesehenen Rahmenkomponente in einem aktiven Bereich der Bildschirmoberfläche jeweils drei GUI-Seiten für jeweils einen Feed oder Tweet nebeneinander angezeigt, wobei die Reihe der GUI-Seiten durch manuelles Wischen über den Touch-Screen des Tablet-Computers über den Bildschirm gezogen werden kann, wodurch randseitige GUI-Seiten aus dem aktiven Bereich herausgeschoben (und somit deaktiviert und ausgeblendet) und andere GUI-Seiten in den aktiven Bereich hineingeschoben (und somit aktiviert und eingeblendet) werden können. Gemäß einer für die Ausführung der Browser-Applikation auf einem Smartphone vorgesehenen Rahmenkomponente wird dagegen zu jedem Zeitpunkt lediglich eine GUI-Seite für jeweils einen Feed oder Tweet angezeigt, wobei zum Wechseln zwischen verschiedenen GUI-Seiten beispielsweise wiederum ein manuelles Wischen über den Touch-Screen oder eine bestimmte Betätigung eines Steuerelements vorgesehen ist.

Die View-Schicht umfasst eine Anzahl von Komponenten, d.h. mindestens eine Komponente, vorzugsweise aber zwei oder mehr Komponenten. Jede Komponente der View-Schicht definiert spezifisch für eine bestimmte Datenart jeweils den graphischen Gehalt einer GUI-Seite zur Anzeige eines Feeds oder zur Anzeige eines Tweets. In einer zweckmäßigen Ausführung umfasst die View-Schicht für jede Datenart, auf die die Browser-Applikation ausgerichtet ist,
- jeweils eine Komponente (exemplarisch als "<Ressource>ListView" bezeichnet), die eine GUI-Seite zur Anzeige eines Feeds definiert, und
- eine weitere Komponente (exemplarisch als "<Ressource>ListItemView" bezeichnet), die eine GUI-Seite zur Anzeige eines Tweets definiert.

Eine entsprechende Browser-Applikation zur ausschließlichen Verwaltung und Bearbeitung von Patientendaten enthält somit in der View-Schicht zwei Komponenten (nämlich "<Patient>ListView" und "<Patient>ListItemView").

Jede Komponente der View-Schicht definiert hierbei insbesondere die Art, das Aussehen und Anordnung der graphischen Elemente der zugehörigen GUI-Seite, insbesondere der Steuerelemente, über die Information an einem Nutzer ausgegeben oder Nutzereingaben und -befehle aufgenommen werden können. Durch die Komponenten der View-Schicht wird dagegen nicht die mit den Steuerelementen verbundene Logik (UI-Logik) festgelegt, da dies den Komponenten der ViewModel-Schicht vorbehalten ist.

Die ViewModel-Schicht umfasst eine Anzahl von Komponenten, von denen jede mindestens einer Komponente der View-Schicht zugeordnet ist. Wie vorstehend angedeutet, implementiert jede Komponente der ViewModel-Schicht die UI-Logik, also die funktionalen Eigenschaften und Befehle für die Steuerelemente der in der zugehörigen Komponente der View-Schicht definierten GUI-Seite. In zweckmäßiger Ausführung umfasst die ViewModel-Schicht zwei Komponenten, von denen eine erste Komponente (exemplarisch als "GenList-ViewModel" bezeichnet) die UI-Logik einer GUI-Seite zur Anzeige eines Feeds implementiert, während die zweite Komponente (exemplarisch als "GenListItemViewModel" bezeichnet) die UI-Logik einer GUI-Seite zur Anzeige eines Tweets implementiert.

Die Model-Schicht umfasst eine Anzahl von Komponenten, also wiederum mindestens eine Komponente. Die oder jede Komponente der Model-Schicht definiert hierbei spezifisch für eine bestimmte Datenart die Struktur der zugehörigen Datensätze, insbesondere die dem Datensatz zugehörigen Variablen.

Die Treiberschicht umfasst ebenfalls eine Anzahl von Komponenten, also wiederum mindestens eine Komponente, vorzugsweise aber zwei oder mehr Komponenten. Jede Komponente der Treiberschicht (nachfolgend als "Treiberkomponente" bezeichnet) ist hierbei dazu eingerichtet, den Datenzugriff der übergeordneten Schichten auf einen bestimmten Datenspeicher zu vermitteln. Die Browser-Applikation lädt Feeds und Tweets und/oder die zugrundeliegenden Datensätze somit ausschließlich über diejenige Treiberkomponente, die auf den jeweiligen Datenspeicher ausgerichtet ist, auf dem die Tweets bzw. Datensätze hinterlegt sind. Der durch die Treiberkomponenten vermittelte Datenzugriff ist hierbei bidirektional und ermöglicht somit sowohl einen (insbesondere asynchronen) Download als auch einen (insbesondere wiederum asynchronen) Upload von Feeds, Tweets und gegebenenfalls Datensätzen durch die Browser-Applikation. Durch die oder jede Treiberkomponente wird eine automatische Datenbindung zwischen dem GUI der Browser-Applikation und dem jeweiligen Datenspeicher hergestellt.

Bei dem Datenspeicher kann es sich um einen internen Datenspeicher, z.B. den Arbeitsspeicher des Nutzergeräts, oder um einen externen Datenspeicher handeln. In letzterem Fall ist der Datenspeicher insbesondere durch einen Cloud-Storage einer Public Cloud gegeben, auf den die zugehörige Treiberkomponente direkt - ohne Inanspruchnahme eines zwischengeschalteten Servers oder Dienstes - zugreift. Verallgemeinernd kann der Datenspeicher im Rahmen der Erfindung allgemein ein beliebiger Teilnehmer eines (drahtlosen oder drahtgebundenen) Computernetzes sein, mit dem medizinische Datensätze bzw. zugehörige Feeds und Tweets ausgetauscht werden können, wobei durch die jeweiligen Treiberkomponente ein beliebiges Datenübertragungsprotokoll (z.B. auch E-Mail, SMS oder Twitter) verwendet werden kann. Sofern die Treiberschicht mehrere Treiberkomponenten umfasst, sind diese vorzugsweise unterschiedlichen Datenspeichern (z.B. Cloud-Storages von mehreren Cloud Vendors) zugeordnet.

Die View-Schicht, die ViewModel-Schicht und die Model-Schicht entsprechen dem Grundprinzip nach dem sogenannten MVVM(Model View ViewModel)-Schema, wie es an sich bei der Programmierung von Applikationen mit graphischer Nutzeroberfläche gängig ist. Ein wesentlicher Unterschied der anmeldungsgemäßen Komponenten-Architektur gegenüber dem gängigen MVVM-Schema besteht hierbei allerdings darin, dass die Komponenten der ViewModel-Schicht generisch, das heißt unabhängig von der speziellen Datenart der verarbeiteten Datensätze sind. Ein und dieselben Komponenten der ViewModel-Schicht können daher unverändert zur Verarbeitung von Datensätzen verschiedener Datenarten (z.B. Patientendaten ebenso wie Aufgabenlisten) oder zugehöriger Feeds und Tweets eingesetzt werden. Die generische Gestaltung der ViewModel-Schicht ermöglicht hierbei eine wesentliche Reduzierung des Herstellungsaufwands für die oder jede Browser-Applikation. Insbesondere wird hierdurch eine vollständig oder zumindest nahezu automatische Generierung der oder jeder Browser-Applikation ermöglicht.

Zudem wird das gewöhnliche MVVM-Modell nach der erfindungsgemäßen Lehre durch zwei weitere Schichten, nämlich die Rahmenschicht und die Treiberschicht erweitert. Durch die zuoberst der Schichtenarchitektur ergänzte Rahmenschicht wird hierbei ermöglicht, einen Großteil der Komponenten der Browser-Applikation bei Anpassung der Applikation an unterschiedliche Nutzergerätearten (PC, Smartphone, Tablet, etc.) unverändert wiederzuverwenden, wodurch der Herstellungsaufwand für die diversifizierten Applikationsvarianten wesentlich reduziert wird. Durch die zuunterst der Schichtenarchitektur ergänzte Treiberschicht mit ihren ggf. mehreren, austauschbaren Treiberkomponenten wird wiederum ein flexibler Einsatz der Browser-Applikation mit unterschiedlichsten Datenspeichern (z.B. dem lokalen Speicher des Nutzergeräts ebenso wie dem Cloud Storage eines oder mehrerer Public Cloud Vendors) ermöglicht. In bevorzugter Ausführung des Systems sind auch die Komponenten der Treiberschicht und/oder die Komponenten der Rahmenschicht generisch, also unabhängig von der Datenart der verarbeiteten Datensätze. Dies ermöglicht eine weitere Reduzierung des Herstellungsaufwands für die oder jede Browser-Applikation und begünstigt ebenfalls eine automatische Generierung der oder jeder Browser-Applikation.

Vorzugsweise implementieren die Komponenten der ViewModel-Schicht eine generische CRUD(Create-Read-Update-Delete)-Funktionalität, insofern als die Komponenten der ViewModel-Schicht Funktionen zum Erzeugen (Create), Lesen (Read), Aktualisieren (Update) und Löschen (Delete) eines Feeds (mit darin enthaltenen Tweets) umfassen, die auf Feeds und Tweets zu Datensätzen beliebiger Datenart angewendet werden können. Mithin können diese Funktionen unverändert auf Feeds und Tweets bzw. Datensätze aller im Rahmen des Systems verwalteten Datenarten angewendet werden. Zweckmäßigerweise wirken die bezüglich eines Tweets definierten Funktionen zumindest zum Teil auch auf den zugehörigen Datensatz. Beispielsweise wird beim Löschen eines Tweets, der einem Patientendatensatz zugeordnet ist, auch dieser Patientendatensatz gelöscht.

Vorzugsweise sind die Komponenten der View-Schicht, der ViewModel-Schicht und der Model-Schicht mehrfach instantiierbar. Jeder - von der Browser-Applikation angezeigten oder im Hintergrund des GUI gehaltenen - GUI-Seite eines Feeds bzw. jeder GUI-Seite eines Tweets ist dabei zweckmäßigerweise jeweils eine eigene Instanz der zugehörigen Komponenten der View-Schicht, der ViewModel-Schicht und der Model-Schicht zugeordnet. Die verschiedenen Instanzen der View-Schicht, der ViewModel-Schicht und der Model-Schicht werden dabei zweckmäßigerweise nebenläufig ausgeführt.

Vorzugsweise ist des Weiteren auch jede Treiberkomponente mehrfach instantiierbar, wobei jeder GUI-Seite auch eine eigene Instanz einer geeigneten Treiberkomponente zugeordnet ist. Auch die verschiedenen Instanzen der Treiberkomponenten werden zweckmäßigerweise nebenläufig ausgeführt.

Die Treiberkomponenten sind vorzugsweise dazu eingerichtet, den Datenzugriff asynchron zu vermitteln. Der Kontrollfluss zwischen den Treiberkomponenten und den Komponenten der übergeordneten Schichten ist somit derart gestaltet, dass letztere bei einer Anfrage an die jeweilige Treiberkomponente nicht auf die Antwort der Treiberkomponente warten.

Um die Browser-Applikation mit besonders geringen Aufwand an unterschiedliche Datenspeicher anpassen zu können, umfasst die Treiberschicht vorzugsweise eine vorgegebene Anwender-Programmier-Schnittstelle (nachfolgend als "Treiber-API" bezeichnet), auf der alle Treiberkomponenten der Treiberschicht basieren.

In einer besonders vorteilhaften Ausführungsvariante umfasst das System ein Applikations-Template (d.h. eine Applikations-Vorlage, in der ein Grundgerüst der Browser-Applikation konfigurierbar vorgegeben ist) sowie einen Applikations-Konfigurator. Der Applikations-Konfigurator, ist dazu eingerichtet, die oder jede Browser-Applikation aus dem Applikations-Template anhand von vorgegebener KonfigurationsInformation automatisch zu generieren. Die KonfigurationsInformation umfasst insbesondere folgende Angaben:
- Angaben zu dem Nutzergerät, auf dem die jeweilige Browser-Applikation ablaufen soll. Diese Angaben umfassen z.B. die Bezeichnung des Gerätetyps, die Bezeichnung des Betriebssystems, etc.
- Angaben zu der oder jeder Datenart der Datensätze sowie zugehörigen Feeds bzw. Tweets, die mittels der zu generierenden Browser-Applikation angezeigt und bearbeitet werden sollen, und/oder
- Angaben zu dem oder jeden Datenspeicher, auf den die jeweilige Browser-Applikation zugreifen soll.

Anhand dieser Angaben konfiguriert der Applikations-Konfigurator das Applikations-Template mit den jeweils geeigneten Komponenten und generiert so die Browser-Applikation.

Der Applikations-Konfigurator kann durch ein eigenständiges, von der Browser-Applikation unabhängiges Software-Programm realisiert sein. Vorzugsweise ist die Funktion des Applikations-Konfigurators aber in die Rahmenkomponente integriert. Vorzugsweise werden hierzu in einem Datenspeicher das Applikations-Template, das beispielsweise als XML-Datei vorliegt, sowie verschiedene Versionen der Rahmenkomponente bereitgestellt, wobei jede Version der Rahmenkomponente auf eine bestimmte Nutzergeräteart und gegebenenfalls einen bestimmten Applikationsstil ausgerichtet ist (die Konfigurationsinformation bezüglich der Art des Nutzergeräts ergibt sich hierbei implizit aus der Wahl der geeigneten Version der Rahmenkomponente). Beim Laden der jeweiligen Rahmenkomponente konfiguriert die Rahmenkomponente dann die Browser-Applikation anhand des Applikations-Templates durch automatisches Nachladen geeigneter Komponenten der untergeordneten Schichten.

Bei dem Datenspeicher, in dem das Applikations-Template und die mindestens eine Rahmenkomponente vorgehalten werden, handelt es sich vorzugsweise um einen Cloud Storage (App Store) einer Public Cloud, aus dem die Rahmenkomponente und das Applikations-Template auf das Nutzergerät heruntergeladen werden können. Das Applikations-Template und/oder die mindestens eine Rahmenkomponente können alternativ aber auch in einem Speicher des Nutzergeräts oder einem mobilen Speichermedium (z.B. einer CD-Rom oder einem USB-Stick) vorgehalten werden.

Nachfolgend werden Ausführungsbeispiele der Erfindung anhand einer Zeichnung näher erläutert. Darin zeigen:
- FIG 1: in einem schematischen Blockschaltbild ein System zur Verwaltung und Bearbeitung von Daten einer medizinischen Einrichtung, mit mehreren Modalitäten und Nutzer- oder Endgeräten (Devices) sowie mit einer mit den Modalitäten und Devices über das Internet verbundenen Public Cloud,

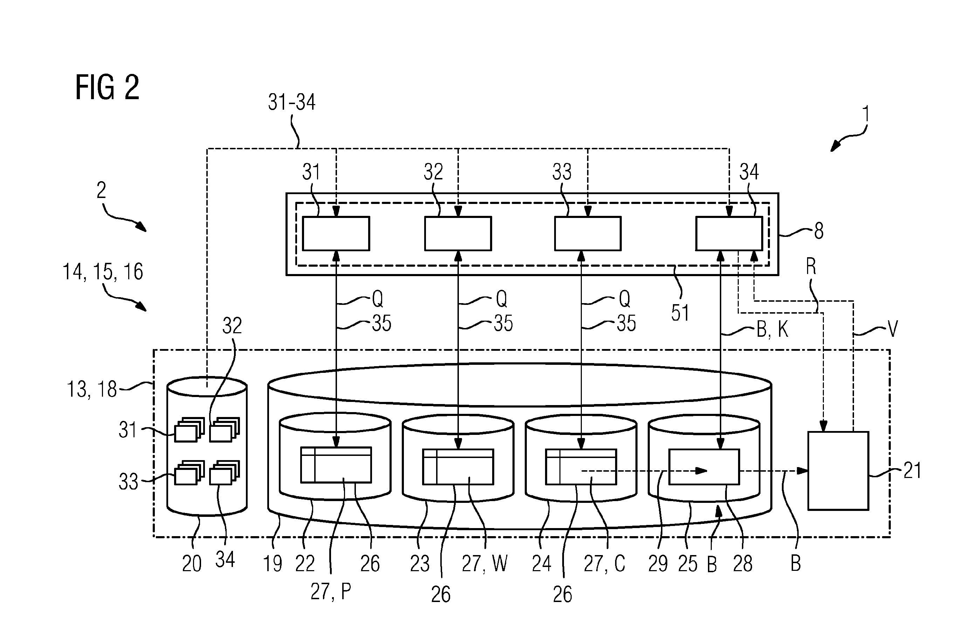

- FIG 2: in einem schematischen Blockschaltbild den softwaretechnischen Aufbau des Systems, wonach das System für verschiedene Arten von Daten der medizinischen Einrichtung in einem Cloud Storage der Public Cloud jeweils spezifisch zugeordnete Speicherbereiche (Hubs) aufweist, nämlich einen Patient-Hub für Patientendatensätze, einen Worklist-Hub für Aufgaben, einen Context-Hub für Kontextdatensätze und einen Image-Hub für Bilddatensätze, und wobei das System auf jedem Device für jeden Hub eine zugeordnete Applikation zum Zugriff auf die in diesem Hub gespeicherten Datensätze enthält, wobei in den drei erstgenannten Hubs jeweils ein Tabellenspeicher (Table Storage) angelegt ist, in dem zu jedem Datensatz des Hubs ein zugeordneter Listeneintrag (Tweet) enthalten ist, und wobei jede Applikation dazu eingerichtet, den Tabellenspeicher des zugehörigen Hubs zu durchsuchen (Browsing) und eine Datensatzliste (Feed) ausgewählter Tweets zu extrahieren und anzuzeigen, und wobei die dem Image-Hub zugeordnete Applikation zur Anzeige (Viewing) der Bilddatensätze eingerichtet ist,
- FIG 3: in schematischer Darstellung eine GUI-Seite einer Benutzeroberfläche (GUI) einer zum Ablauf auf einem Smartphone vorgesehenen Browser-Applikation, die zur Anzeige eines Feeds des jeweils zugeordneten Hubs dient,
- FIG 4: in Darstellung gemäß FIG 3 eine weitere Seite der Benutzeroberfläche der Browser-Applikation, die zur Anzeige eines Tweets des zugehörigen Hubs dient,
- FIG 5: in Darstellung gemäß FIG 3 eine zum Ablauf auf einem Personal-Computer, Notebook oder Tablet-Computer vorgesehene Variante der Browser-Applikation, bei der mehrere GUI-Seiten der Benutzeroberfläche gleichzeitig darstellbar sind,
- FIG 6: in einem schematischen Blockdiagramm die Komponenten-Architektur der Browser-Applikation gemäß FIG 3 und 4 oder der Browser-Applikation gemäß FIG 5, und
- FIG 7: in einem schematischen Blockschaltbild die Browser-Applikation gemäß FIG 5.

Einander entsprechende Teile und Größen sind in allen Figuren stets mit gleichen Bezugszeichen versehen.

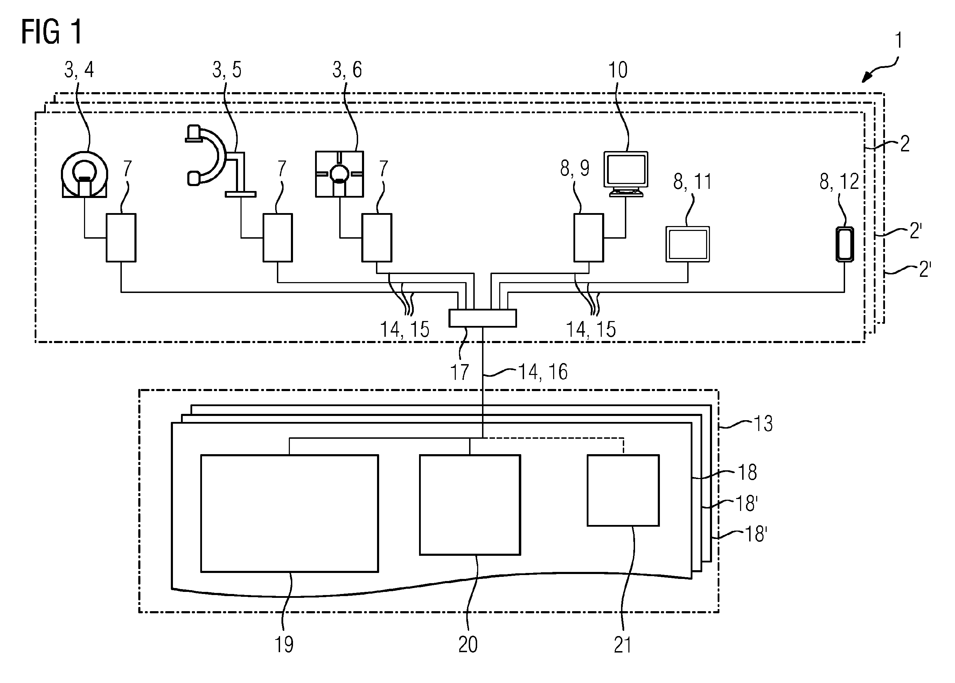

FIG 1 zeigt in grober schematischer Vereinfachung ein System 1 zur Verwaltung und Bearbeitung von medizinischen Daten einer medizinischen Einrichtung 2, bei der es sich beispielsweise um eine Klinik handelt.

Hardwareseitig umfasst das System 1 eine Anzahl von Modalitäten 3, also bildgebenden, medizinischen Untersuchungsgeräten der Einrichtung 2. So umfasst das System 1 in der beispielhaften Darstellung gemäß FIG 1 einen Computertomographen 4, ein C-Bogengerät 5 und einen Magnetresonanztomographen 6. Jeder Modalität 3 ist ein (Steuer- und Auswerte-)Rechner 7 zugeordnet.

Weiterhin umfasst das System 1 hardwareseitig eine Anzahl von Nutzer- oder Endgeräten (nachfolgend Devices 8) der Einrichtung 2, die zur Anzeige und Bearbeitung von Daten dienen. In dem vereinfachten Beispiel gemäß FIG 1 umfassen die Devices 8 einen Personalcomputer 9 mit angeschlossenem Bildschirm 10, ein Tablet 11 sowie ein Smartphone 12.

Als außerhalb der Einrichtung 2 angeordneten Bestandteil umfasst das System 1 eine Public Cloud 13. Als Public Cloud 13 wird im Rahmen des System 1 beispielsweise der von der Firma Microsoft unter der Bezeichnung "Windows Azure" angebotene Dienst genutzt. Abweichend hiervon können im Rahmen der Erfindung allerdings auch eine andere Public Cloud oder eine Kombination mehrerer Public Clouds (gegebenenfalls auch von unterschiedlichen Anbietern) als Public Cloud 13 herangezogen werden.

Die Public Cloud 13 ist über ein (Datenübertragungs-)Netz 14 mit den einrichtungsinternen Komponenten des Systems 1, also den Modalitäten 3 und Devices 8 verbunden. Diese Netzverbindung ist innerhalb der Einrichtung 2 durch ein Intranet 15 der Einrichtung 2 gebildet, das beispielweise als sogenanntes Local Area Network (LAN) auf Basis kabelgebundener Ethernet-Technologie und/oder als kabelloses Wireless Local Area Network (WLAN) aufgebaut ist. Außerhalb der Einrichtung 2 ist das Netz 14 durch das Internet 16 gebildet. An der Schnittstelle zwischen dem Intranet 15 und Internet 16 ist in üblicher Weise eine Firewall 17 angeordnet.

Die von der Public Cloud 13 im Rahmen des Systems 1 zur Verfügung gestellten Dienste sind durch einen als Subscription 18 bezeichneten Nutzungsvertrag festgelegt. Die Subscription 18 regelt dabei, welche Hardware- und Softwarebestandteile der Public Cloud 13 den einrichtungsinternen Komponenten des Systems 1 zugänglich sind. Mit dem Begriff Subscription 18 wird daher nachfolgend derjenige Teil der Public Cloud 13 bezeichnet, der der Einrichtung 2 im Rahmen des Systems 1 exklusiv zugeordnet ist. Andere Bereiche der Public Cloud 13 können - wie in FIG 1 angedeutet - im Rahmen weiterer Subscriptions 18' weiteren medizinischen Einrichtungen 2' zugewiesen sein. Jede Einrichtung 2,2' hat hierbei ausschließlich Zugang zu den ihr gemäß ihrer Subscription 18,18' zugewiesenen Daten und Diensten, nicht aber zu den Daten und Diensten anderer Einrichtungen 2 bzw. 2'. Die Public Cloud 13 ist in diesem Sinne "mandantenfähig" (multi-tenant). "Mandant" (Tenant) einer Subscription 18,18' ist vorzugsweise ein Krankenhaus, eine Arztpraxis oder eine sonstige medizinische Einrichtung. Grundsätzlich ist aber auch vorstellbar, Subscriptions 18,18' an kleinere Einheiten bzw. Personengruppen bis hin zu einzelnen Patienten zu vergeben.

Gemäß der Subscription 18 sind dem System 1 der Einrichtung 2 innerhalb der Public Cloud 13
- ein nachfolgend als Cloud Storage 19 bezeichneter Datenspeicher,
- ein nachfolgend als App Store 20 bezeichneter Speicher für Applikationen (Anwendungssoftware) sowie
- Cloud-Compute-Dienste 21

zur Verfügung gestellt. Der Cloud Storage 19 dient zur persistenten Speicherung der Daten der Einrichtung 2. Der App Store 20 stellt Applikationen, Treiber und sonstige Peripheriesoftware wie beispielsweise Konfigurationsdateien und Vorlagen (Templates) zur Verfügung, die von den einrichtungsinternen Komponenten des Systems 1, also den Modalitäten 3 und den Devices 8 heruntergeladen werden können und im Betrieb des Systems 1 auf den Modalitäten 3 (genauer gesagt den zugeordneten Rechnern 7) bzw. auf den Devices 8 ablaufen. Die Cloud-Compute-Dienste 21 werden im Rahmen des Systems lediglich optional für alle Rechenoperationen herangezogen, die nicht auf den Modalitäten 3 oder den Devices 8 selbst vorgenommen werden. Letzteres betrifft insbesondere die rechenleistungsintensive Aufbereitung von 3D-Bilddaten (Volumendaten) der jeweiligen Modalität 3 für die Speicherung (Preprocessing) und/oder die Ableitung von gerenderten Ansichten V (Bildszenen oder Scene-Graphs) für die zweidimensionale Visualisierung (Bildsynthese, Volume Rendering) solcher Volumendaten.

Die im Rahmen der Einrichtung 2 zu verwaltenden und zu bearbeitenden Daten umfassen gemäß FIG 2 Patientendatensätze P, Aufgaben W, Kontextdatensätze C und Bilddatensätze B, wobei diese Daten im Einzelnen jeweils die vorstehend näher erläuterten Inhalte haben. Bilddatensätze B können hierbei medizinische Bilddaten oder Volumendaten oder eine sonstige Bild-, Ton-, Graphik- oder Textinformation (Photos, Videos, Dokumente) enthalten.

Gemäß FIG 2 ist für jede dieser verschiedenen Datenarten (Ressourcen) innerhalb des Cloud Storage 19 jeweils ein separater Speicherbereich angelegt, der nachfolgend als (Ressource-)Hub bezeichnet ist. Im Einzelnen sind in dem Cloud Storage 19 somit
- ein (Patient-)Hub 22 zur Speicherung der Patientendatensätze P,
- ein (Worklist-)Hub 23 zur Speicherung der Aufgaben W,
- ein (Context-)Hub 24 zur Speicherung der Kontextdatensätze C sowie
- ein (Image-)Hub 25 zur Speicherung der Bilddatensätze B angelegt.

Jeder der Hubs 22-24 enthält jeweils einen Tabellenspeicher (nachfolgend als Table Storage 26 bezeichnet).

Im Rahmen des Systems 1 wird für jeden gespeicherten Datensatz in dem Table Storage 26 des jeweils zugeordneten Hubs 22-24 ein Listeneintrag angelegt, der nachfolgend als Tweet 27 bezeichnet ist.

Bei Datensätzen, die ihrer Natur nach aus vergleichsweise wenigen, diskreten Angaben bestehen, ist dabei der gesamte Datensatz oder zumindest ein Teil desselben als Tweet 27 im Table Storage 26 des jeweiligen Hubs 22-24 gespeichert. Dies betrifft im Beispiel gemäß FIG 2 die Patientendatensätze P, die Aufgaben W, sowie die Kontextdatensätze C. Anstelle des Table Storage 26 kann der Worklist-Hub 23 zur Speicherung der Aufgaben W nach einem First-in-first-out-Prinzip allerdings auch einem sogenannten Queue-Storage aufweisen.

Zur Speicherung der Bilddatensätze B, die in der Regel im Wesentlichen aus einem größeren und nicht-alphanumerischen Datenblock gebildet sind, ist im Image Hub 25 anstelle eines Table-Storage 26 ein sogenannter Blob-Storage 28 vorgesehen, in dem die Bilddatensätze B als "Binary Large Objects (BLOB)" in nicht weiter strukturierter Form gespeichert sind. Zu den in dem Image Hub 25 gespeicherten Bilddatensätzen B ist in dem Kontext Hub 24 jeweils ein Kontextdatensatz C in Form eines Tweets 27 hinterlegt. Dieser Tweet 27 enthält zumindest einen URL 29 (Uniform Ressource Locator), der den Speicherort des Bilddatensatzes B im Blob-Storage 28 des Image Hubs 25 bezeichnet.

Für jeden der Hubs 22-25 wird im App Store 20 eine Applikation 31-34 zur Verfügung gestellt, die jeweils selektiv zur Darstellung und zur Bearbeitung der in dem jeweils zugeordneten Hub 22-25 gespeicherten Datensätze dient. Jede der Applikationen 31-34 repräsentiert somit den zugeordneten Hub 22-25 auf der Ebene des Devices 8. Die Applikationen 31-34 sind daher - in Anlehnung an die Ressource-Hubs - auch als "Applications-Hubs" bezeichnet.

Im Einzelnen umfassen die im App Store 20 zur Verfügung gestellten Applikationen somit
- eine Applikation 31 zur Verwaltung und Bearbeitung der in dem Patient-Hub 22 gespeicherten Patientendatensätze P,
- eine Applikation 32 zur Verwaltung und Bearbeitung von Aufgaben W,
- eine Applikation 33 zur Verwaltung und Bearbeitung von Kontextdatensätzen C, sowie
- eine Applikation 34 zur Anzeige und Bearbeitung der in dem Image-Hub 25 gespeicherten Bilddatensätze B.

Die Applikationen 31-34 können aus dem App Store 20 auf jedes der Devices 8 heruntergeladen und dort zur Ausführung gebracht werden. Sofern das System 1 - wie in FIG 1 dargestellt - unterschiedliche Arten von Devices (z.B. Personal-Computer 9, Tablet-Computer 11 und/oder Smartphones 12) umfasst, werden in dem App Store 20 jeweils verschiedene Versionen der Applikationen 31-34 zur Verfügung gestellt, die zum Ablauf auf dem jeweiligen Device 8 angepasst sind. Vorzugsweise werden die Applikationen 31-34 und deren Varianten allerdings nicht in vorgefertigtem Zustand im App Store 20 vorgehalten. Vielmehr ist im App Store 20 zumindest für die funktionsähnlichen (Browser-)Applikationen 31-33 eine gemeinsames Applikations-Template in Form einer XML-Datei hinterlegt, aus dem die jeweilige Applikation bei Anforderung durch eines der Devices 8 mittels eines Applikations-Konfigurators anhand von vorgegebener Konfigurationsinformation automatisch generiert wird.

Beispielsweise werden die Applikationen 31-34 zum Ablauf auf dem Tablet-Computer 11 und dem Smartphone 12 in Form einer an den jeweiligen Gerätetyp angepassten App zur Verfügung gestellt, während die für den Personal-Computer 9 zur Verfügung gestellte Versionen der Applikationen 31-34 für den Ablauf in einem Web-Browser konzipiert sind.

Im Vergleich zu einer herkömmlichen IT-Struktur für die Verwaltung von medizinischen Daten übernehmen der Patient Hub 22 und die zugeordnete Applikation 31 aus Sicht des Nutzers etwa die Funktion eines herkömmlichen Krankenhaus-Informationssystems (HIS). Insbesondere sind die gesamten personenbezogenen (und datenschutzrelevanten) Patientendaten in dem Patient-Hub 22 aufgenommen, der somit für den einzelnen Patienten eine elektronische Patientenakte abbildet. Die übrigen Hubs 23-25 enthalten vorzugsweise keine Information, die aus sich heraus einem bestimmten Patienten zuordenbar wäre (Privacy Information). Der Worklist-Hub 23 und die zugeordnete Applikation 32 stellen sich dem Nutzer im Wesentlichen als Entsprechung zu einem herkömmlichen Radiologie-Informationssystems (RIS) dar, indem sie die innerhalb der Einrichtung 1 zur Durchführung anstehenden Aufgaben auflisten. Der Context-Hub 24 und die zugeordnete Applikation 33 übernehmen aus Nutzersicht etwa die Funktion eines herkömmlichen PACS, indem dort die zur Auffindung und Verarbeitung von Bilddatensätzen B erforderliche Information hinterlegt und recherchierbar ist. Der Image-Hub 25 und die zugeordnete Applikation 34 übernehmen schließlich etwa die Funktion eines herkömmlichen AV-Systems.

Obwohl die in den Hubs 22-25 und den zugeordneten Applikationen 31-34 enthalten Funktionen hochgradig miteinander verknüpft sind, ist jeder der Hubs 22-25 zusammen mit der jeweils zugeordneten Applikation 31-34 auch unabhängig von den jeweils anderen Hubs 22-25 und deren Applikationen 31-34 betreibbar. So kann beispielsweise auf einen in dem Image-Hub 25 enthaltenen Bilddatensatz B auch ohne den zugehörigen Kontextdatensatz C des Context-Hubs 24 zugegriffen werden, wenn der dem Image-Hub 25 zugeordneten Applikation 34 der URL 29 des anzuzeigenden Bilddatensatzes B auf andere Weise verfügbar gemacht wird, z.B. per E-Mail, SMS oder Twitter. Ferner können die Patientendaten P auch außerhalb der Public Cloud 13 vorgehalten werden, da der Patient-Hub 22 für die Funktion der übrigen Hubs 23-25 und der zugeordneten Applikationen 32-34 nicht erforderlich ist.

Die Applikationen 31-33 dienen ausschließlich zum Auffinden bestimmter Daten aus dem Datenspeicher nach Maßgabe vorgegebener Kriterien (Browsing). Sie dienen insbesondere nicht zur Anzeige von großformatigen medizinischen Bilddaten (Viewing). Allenfalls können sie sogenannte Stamp-Images, also stark verkleinerte Abbilder von medizinischen Bilddatensätzen als charakteristische Information innerhalb ihrer Listen darstellen. Diese Browsing-Applikationen sind nun so konzipiert, dass ihre Listen allesamt aus dem REST-Standard des Cloud-Storage 19 gespeist werden, wodurch ein eigener Server-Service hierfür entfallen kann.

Die dem Context-Hub 24 zugeordnete Browsing-Applikation 33 verwaltet eine Liste (Feed) von Einträgen (Tweet) die zusammen z.B. die Untersuchungen (Study) eines Patienten darstellen, und deren jeweilige Inhalte direkt an die Viewing-Applikation 34 des Image-Hub 25 weitergegeben werden können. Insbesondere sind die Applikation 33 (und auch der zugehörige Context-Hub 24) aber unabhängig von der Viewing-Applikation 34 (und auch dem zugehhörigen Image-Hub 25). Die Applikation 33 stellt quasi das Äquivalent eines PACS-Browser dar, der sich aber nicht des Viewings (also der Bildanzeige) annimmt, sondern Verweise auf anzuzeigende Bilddatensätze jeweils in Form eines Tweets weitergeben kann.

Die dem Worklist-Hub 23 zugeordnete Applikation 32 verwaltet eine Liste (Feed) von Einträgen (Tweet) die zusammen z.B. die Aufträge (Procedures) eines "Mandanten" (Tenants) darstellen, und deren jeweilige Inhalte direkt an die dem Context-Hub 23 zugeordnete Applikation 33 weitergegeben werden können. Insbesondere sind die Applikation 32 (und auch der zugehörige Worklist-Hub 23) aber unabhängig von der Applikation 33 (und auch dem zugehörigen Context-Hub 24). Die Applikation 32 stellt quasi das Äquivalent eines RIS-Browsers dar, der sich aber nicht der Kontextinformation annimmt, sondern Verweise hierauf jeweils in Form eines Tweets weitergeben kann. Abstrakter formuliert könnte man sagen, dass die Applikation 32 eine Liste von Verweisen auf Kontextinformation verwaltet, die mehr kurzfristiger Natur sind, und typischerweise in einem engen Zeitraum (z.B. innerhalb eines Tages) abzuarbeiten sind.

Die dem Patient-Hub 22 zugeordnete Applikation 31 verwaltet eine Liste (Feed) von Einträgen (Tweet) die zusammen z.B. die Patienten-Akten eines Tenants darstellen, und deren jeweilige Inhalte direkt an die Applikation 32 weitergegeben werden können. Insbesondere sind die Applikation 31 (und auch der Patient-Hub 22) aber unabhängig von der Applikation 32 (und auch dem zugehörigen Worklist-Hub 23). Die Applikation 31 stellt dabei quasi das Äquivalent eines HIS/EPR-Browsers dar, der sich aber nicht der Worklist oder der Kontextinformation annimmt, sondern Verweise hierauf jeweils in Form eines Tweets weitergeben kann. Abstrakter formuliert könnte man sagen, dass die Applikation 31 eine Liste von Verweisen auf Kontextinformation verwaltet, die mehr langfristiger, permanenter Natur sind, und typisch die Kontexte darstellen, die ein Patient während seines Lebens (durch verschiedene Untersuchengen) erfährt, und die als Referenz-Untersuchungen zu verwenden sind.

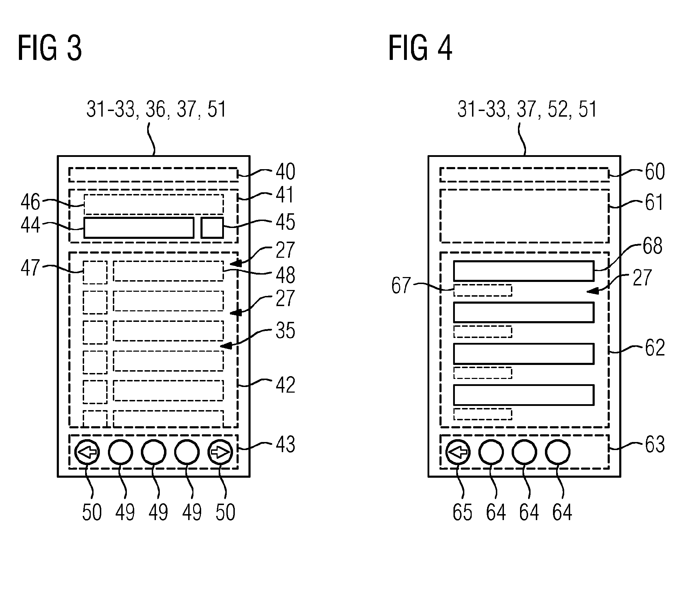

Bei der dem Patient-Hub 22 zugeordneten Applikation 31 handelt es sich um eine Browser-Applikation, die den Table Storage 26 des Patient-Hubs 22 nach Tweets 27 durchsucht, die einem bestimmten, von einem Nutzer vorgebbaren Suchbegriff entsprechen. Von dem Patient-Hub 22 wird hierbei auf eine Suchanfrage Q (FIG 2) der Applikation 31 hin eine nachfolgend als Feed 35 bezeichnete Datensatzliste, die die der Suchanfrage Q entsprechenden Tweets 27 enthält, an die Applikation 31 zurückgeliefert. Die Applikation 31 zeigt den erhaltenen Feed 35 auf dem Device 8 auf einer in FIG 3 exemplarisch dargestellten GUI-Seite 36 einer graphischen Benutzeroberfläche 37 (GUI) an.

In der exemplarischen Ausführung gemäß FIG 3 weist die GUI-Seite 36 im Wesentlichen vier Anzeigebereiche unterschiedlicher Funktion auf, nämlich eine Kopfzeile 40, ein Suchfeld 41, ein Anzeigefeld 42 und eine Befehlsleiste 43. In der Kopfzeile 40 ist der Hub 22-24 namentlich benannt, dem die Applikation 31 zugeordnet ist. Im Beispiel der Applikation 31 ist die Kopfzeile 40 somit z.B. mit dem Wort "Patient Hub" beschriftet. Des Weiteren ist in der Kopfzeile 40 vorzugsweise die Einrichtung 2 benannt, der das System 1 zugeordnet ist.

Das Suchfeld 41 enthält eine Eingabezeile 44, über die ein alphanumerischer Suchbegriff eingegeben werden kann, sowie eine Schaltfläche 45, durch deren Betätigung von einem Nutzer eine Suchanfrage Q auf Basis des zuvor in der Eingabezeile 44 eingegebenen Suchbegriffs gestartet werden kann.

Zusätzlich oder alternativ enthält das Suchfeld 41 ein Vorauswahlfeld 46, über das ein oder mehrere voreingestellte Suchbegriffe durch Antippen (im Falle eines berührungssensitiven Bildschirms) oder Anklicken mit einem gegebenenfalls vorhandenen Zeigergerät ausgewählt werden können.

Bei den über das Vorauswahlfeld 46 generierbaren Suchbegriffen handelt es sich insbesondere um häufig verwendete Suchschemata. Beispielsweise ermöglicht das Vorauswahlfeld 46 im Falle der mit dem Patient-Hub 22 korrespondierenden Applikation 31 eine Vorauswahl dahingehend, ob
- alle Tweets 27 ("all") oder
- nur solche Tweets 27 zu Patienten, für die Untersuchungen angesetzt sind ("scheduled") oder
- nur solche Tweets 27 zu Patienten, bei denen die zurückliegenden Leistung bereits abgerechnet wurden ("billed")
als Feed 35 zurückgeliefert werden sollen.

Optional ist vorgesehen, dass bei Generierung eines Suchbegriffs über das Vorauswahlfeld 26 eine alphanumerische Entsprechung dieses Suchbegriffs automatisch in der Eingabezeile 44 angezeigt wird.

Im Anzeigefeld 42 wird der aufgrund einer Suchabfrage Q zurückgelieferte Feed 35 angezeigt. Das Anzeigefeld 42 enthält somit in Form einer Liste diejenigen Tweets 27, die aufgrund der Suchanfrage Q der Applikation 31 in dem Patient-Hub 22 gefunden wurden. Zu jedem Tweet 27 werden beispielsweise ein verkleinertes Bild 47 und/oder ein Schlagwort 48 angezeigt. So werden bei der dem Patient-Hub 22 zugeordneten Applikation 31 zu jedem anzeigten Tweet 27 vorzugsweise ein verkleinertes Bild des Patienten sowie der Name des Patienten und optional eine Patientenidentifikationsnummer angezeigt. Die Suchanfrage Q, und der aufgrund dieser zurückgelieferte Feed 35 können von einem Nutzer interaktiv zur Laufzeit der Applikation 31-33 geändert werden.

Die Suche wird basierend auf einer Syntax gemäß REST bzw. OData (Open Data Protocoll Standard) definiert. REST (Representational State Transfer) bezeichnet dabei ein Programmierparadigma für Internet-Anwendungen, das folgenden Prinzipien genügt:
- Jedem angebotenen Dienst ist eine eindeutige Adresse (Uniform Ressource Locator, kurz: URL) zugewiesen.
- Der unter einer Adresse zugängliche Dienst kann unterschiedliche Erscheinungsformen (Repräsentationen) haben.
- Es wird ein zustandsloses (stateless) Protokoll verwendet, das es ermöglicht, verschiedene Anfragen stets unabhängig voneinander zu behandeln.
- Auf alle Ressourcen muss eine vorgegebene Anzahl von Operationen (nämlich insbesondere die vier Operationen GET, POST, PUT, DELETE) mit definierten Eigenschaften anwendbar sein.
- Sowohl für Anwendungsinformationen als auch für Zustandsveränderungen wird multimedialer Hypertext (Hypermedia) benutzt.

Die Repräsentation erfolgt dabei vorzugsweise gemäß den Internet-Standards XML (Extensible Markup Language), JSON (Java Script Object Notation) und/oder ATOM. ATOM ist hierbei als Überbegriff verwendet für das Atom Syndication Format (ASF), das ein XML-Format für den Austausch von Nachrichten darstellt, sowie das ATOM Publishing Protocol (APP), das eine Programmierschnittstelle zur Erzeugung und Bearbeitung von Webinhalten darstellt.

Die Suchfunktion ist grundsätzlich ähnlich zu einer WHERE-Klausel nach dem SQL-Standard. Sie ist jedoch im Gegensatz zu der letzteren nicht von einer statischen Re-Indexierung abhängig. Insbesondere benötigen die Tabellen eines Hubs keine Index-Elemente für die Suche, weshalb auch keine Re-Indexierung erforderlich ist, und entsprechend auch keine eigenen Daten-Server oder Datenbank-Server vorgehalten werden müssen.

Durch einfaches Antippen oder Anklicken eines im Anzeigefeld 42 angezeigten Tweets 27 kann dieser Tweet 27 von einem Nutzer ausgewählt werden.

Die Befehlsleiste 43 enthält eine Anzahl von Schaltflächen 49 und 50, durch deren Betätigung von einem Nutzer elementare Operationen zur Steuerung der Benutzeroberfläche 37 und zum Verwalten und Bearbeiten des angezeigten Feeds 35 vorgenommen werden können.

Die Applikation 31 ist in der in FIG 3 dargestellten Version für die Darstellung auf dem Smartphone 12 optimiert. Dabei wird auf dem Smartphone 12 zu jedem Zeitpunkt stets nur eine einzelne GUI-Seite 36 mit einem einzelnen Feed 35 angezeigt. Allerdings können mehrere Instanzen der GUI-Seite 36 parallel zueinander generiert werden, wobei diese Instanzen im Hintergrund der Anzeige nebenläufig auf dem Smartphone 12 ausgeführt werden. Somit können insbesondere mehrere Suchanfragen Q parallel zueinander abgearbeitet werden. Die GUI-Seiten 36 laufen in einem generischen Rahmen 51 (oder Container) ab. Dieser Rahmen 51 (und damit die gesamte Applikation 31) können beispielsweise als separate Applikation oder als Plug-in für einen Web-Browser implementiert sein. Alternativ kann die Applikation 31 jedoch auch zum unmittelbaren Ablauf in einem Web-Browser ausgebildet sein, z.B. als HTML5-Applikation.

Die Schaltflächen 50 der Befehlsleiste 43 ermöglichen hierbei einen Wechsel zwischen den laufenden GUI-Seiten 36, indem bei Betätigung einer der Schaltflächen 50 die angezeigte GUI-Seite 36 aus der Nutzeroberfläche 37 ausgeblendet und eine andere GUI-Seite 36 angezeigt wird.

Die weiteren Schaltflächen 49 der Befehlszeile 43 sind insbesondere folgenden CRUD-Funktionen zugeordnet:
- "Create": Auf Betätigung der entsprechenden Schaltfläche 49 hin werden eine neue GUI-Seite 36 der Benutzeroberfläche 37 erzeugt.
- "Delete": Auf Betätigung der entsprechenden Schaltfläche 49 hin werden die angezeigte GUI-Seite 36 der Benutzeroberfläche 37 beendet,
- "Read": Auf Betätigung der entsprechenden Schaltfläche 49 hin wird eine - in FIG 4 schematisch dargestellte - GUI-Seite 52 der Benutzeroberfläche 37 geöffnet, in der der Dateninhalt des ausgewählten Tweets 27 zur bloßen Ansicht oder zur Veränderung angezeigt wird.

Die GUI-Seite 52 umfasst gemäß FIG 4 ebenfalls vier Bereiche, nämlich eine Kopfzeile 60, ein Überschriftenfeld 61, ein Anzeigefeld 62 sowie eine Befehlsleiste 63.

Die Kopfzeile 60 weist hierbei - analog zu der Kopfzeile 40 der GUI-Seite 36 - den zugeordneten Hub 22-24 und die Einrichtung 2 aus. Das Überschriftenfeld 61 kennzeichnet die Art des angezeigten Datensatzes und enthält somit im Falle der Applikation 31 beispielsweise das Wort "Patient" (oder "Patient Entity").

In dem Anzeigefeld 62 wird der Inhalt des ausgewählten Tweets 27 - im Falle der Applikation 31 somit der Inhalt des (Patienten-)Datensatzes - dargestellt. In diesem Fall werden in dem Anzeigefeld 62 insbesondere die Feldnamen 67 des Patienten-Datensatzes (zum Beispiel "Vorname", "Nachname", "Patienten-ID", "Alter", ...) zusammen mit dem jeweiligen Feldinhalt 68 (zum Beispiel also dem konkreten Vornamen, Nachnamen, der konkreten Patienten-ID bzw. dem konkreten Alter des Patienten dargestellt).

Die Befehlszeile 63 enthält Schaltflächen 64, denen insbesondere folgende CRUD-Funktionen zugewiesen sind:
- "Create": Auf die Betätigung der entsprechenden Schaltfläche 64 hin wird eine neue Instanz der GUI-Seite 52 zur Anlage eines neuen Tweets 27 und somit eines neuen (Patienten-)Datensatzes P erzeugt.
- "Update": Auf die Betätigung der entsprechenden Schaltfläche 64 hin werden die - gegebenenfalls geänderten - Feldinhalte 68 gespeichert, indem die Applikation 31 den angezeigten Tweet 27 unmittelbar im Table Storage 26 des Patient-Hubs 22 mit den geänderten Angaben überschreibt.
- "Delete": Auf die Betätigung der entsprechenden Schaltfläche 64 hin wird der angezeigte Tweet 27 durch die Applikation 31 unmittelbar im Table Storage 26 des Patient-Hubs 22 gelöscht; die angezeigte GUI-Seite 52 wird geschlossen.

Darüber hinaus enthält die Befehlszeile 63 eine Schaltfläche 65, auf deren Betätigung hin die Applikation 31 die angezeigte GUI-Seite 52 ohne Veränderung des Tweets 27 schließt und die zugrundeliegende GUI-Seite 36 erneut anzeigt.

Die Befehlszeilen 43 und 63 können weitere Schaltflächen für Befehle aufweisen. Ebenso können auch eine oder mehrere der anhand der Schaltflächen 49, 50, 64 und 65 beschriebenen Funktionen einem der gegebenenfalls vorhandenen elektromechanischen Tasten des Smartphones 12 zugeordnet sein. Die jeweilige Schaltfläche ist in diesem Fall vorzugsweise aus der Befehlszeile 43 entfernt.

Bei den Applikationen 32 und 33 handelt es sich ebenfalls um Browser-Applikationen, die hinsichtlich ihres Aufbau und der Funktionsweise der Applikation 31 entsprechen. Insbesondere weist auch die jeweilige Benutzeroberfläche 37 der Applikationen 32 und 33 jeweils die anhand der FIG 3 und 4 beschriebenen GUI-Seiten 36 und 52 auf.

Allerdings greifen die Applikationen 32 und 33 auf den Worklist-Hub 23 bzw. auf den Context-Hub 24 zu, und sind entsprechend angepasst. So enthalten insbesondere
- die im Rahmen der Applikation 32 angezeigten Tweets 27 Angaben zu einer bestimmten Aufgabe W (beispielsweise Angaben zu dem Einstellungsdatum der Aufgabe, der mit der Aufgabe betreuten Abteilung, sowie Angaben zu der Aufgabe selbst und dem Taskflow, in den diese Aufgabe gegebenenfalls eingebunden ist), und
- die im Rahmen der Applikation 33 angezeigten Tweets 27 den Inhalt jeweils eines Kontext-Datensatzes C, insbesondere Angaben zum Dokumententyp, Speicherort, Datenformat sowie optional Aufnahmeparameter zu einem Bilddatensatz B.

In einer (nicht näher dargestellten) alternativen Ausführung sind zwei oder sogar alle drei Applikationen 31 bis 33 zu einer übergreifenden Applikation zusammengefasst. Diese übergreifende Applikation umfasst einen gemeinsamen Rahmen 51, in dem mehrere GUI-Seiten 36 und/oder 52 ablaufen, die verschiedenen Ressource-Hubs zugeordnet sind und somit zur Anzeige von Feeds 35 bzw. Tweets 37 unterschiedlicher Datenarten dienen. Bei den Applikationen 31-33 ist der Rahmen 51 insbesondere so gestaltet, dass er mit einer oder mehreren der einzelnen Browsing-Applikationen 31-33 konfigurierbar ist. Wie an den Code-Beispiel im Anhang aufgezeigt, sind der Rahmen 51 und Browsing-Applikationen 31-33 vorzugsweise als Container-App und Sub-Apps in einer einzigen Device-Applikation realisiert, die auch in einem Web-Browser als HTML5-Applikation ablaufen kann.

Die Kommunikation zwischen jedem der Hubs 22-25 und der jeweils zugewiesenen Applikation 31-34 ist - wie vorstehend angedeutet - so gestaltet, dass sie mit dem im Internet-Datenverkehr gebräuchlichen REST(Representational State Transfer)-Prinzip kompatibel ist. Insbesondere genügen die zwischen den Hubs 22-25 und der jeweiligen Applikation 31-34 ausgetauschten Daten den Standards XML, JSON und ATOM. Bilddaten B sind im Image-Hub 25 insbesondere im JPEG-Format hinterlegt, Volumendaten vorzugsweise im DICOM-Format. Die gegebenenfalls von der Public-Cloud 13 an die Applikation 34 gelieferten Ansichten V (Scene-Graphs) von Volumendaten werden vorzugsweise im JPEG-Format erstellt. Zusätzlich oder alternativ können im Image-Hub 25 aber auch Bilddaten und Dokumente in anderen Datenformaten, z.B. webm, mp4, pdf oder dz abgespeichert sein.

Jede der Applikationen 31-33 ist dazu eingerichtet, Feeds 35 auf Anweisung des Nutzers aus dem zugehörigen Hub 22-24 in einen lokalen Speicher des Device 8 zu externalisieren, so dass dieser Feed 35 und die darin enthaltenen Tweets 27 durch die Applikation 31-33 auch ohne Netzanbindung mit der Public Cloud 13 angezeigt und/oder bearbeitet werden können. Weiterhin ist jede Applikation 31-33 dazu eingerichtet, auf entsprechenden Befehl des Nutzers externalisierte Feeds 35 oder Tweets 27 an eine andere Instanz derselben oder einer anderen Applikation 31-34 zu übermitteln. Für die geräteinterne Übermittlung der Feeds 35 oder Tweets 27 ist im Speicher des Device 8 ein als "Exchangeboard" bezeichneter lokaler Speicherbereich eingerichtet, auf den alle Instanzen der Applikationen 31-34 gemeinsam zugreifen. Zum Austausch über das Exchangeboard werden die auszutauschenden Feeds 35 oder Tweets 27 in XML/JSON-Files serialisiert, die im lokalen Speicher des Devices 8 abgelegt und anschließend wieder deserialisiert werden. Zum Austausch von Feeds 35 oder Tweets 27 zwischen verschiedenen Devices 8 weisen die Applikationen 31-34 bevorzugt Schnittstellen zu Twitter oder einem E-Mail-Programm auf.

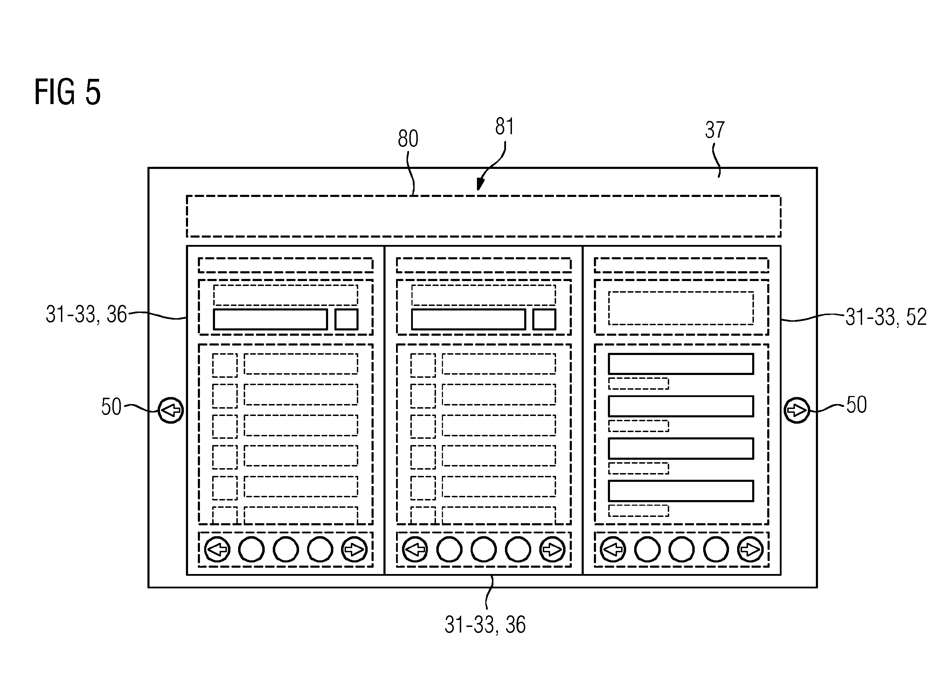

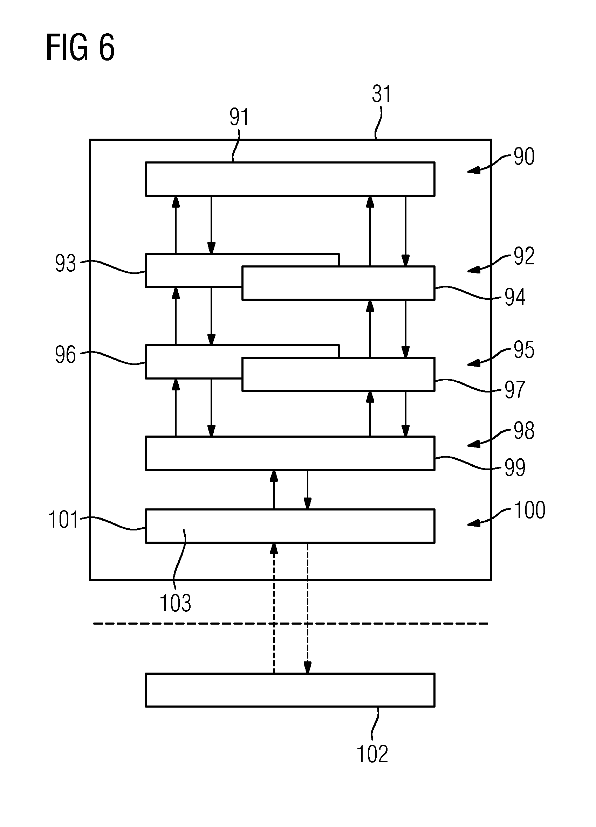

FIG 5 zeigt eine Variante der Applikationen 31-33, bei der die Benutzeroberfläche 37 für die Anzeige auf einem großformatigen Bildschirm, beispielsweise dem Bildschirm 10 des Personal-Computers 9 oder dem Bildschirm des Tablet-Computers 11 optimiert ist. Die Benutzeroberfläche 37 gemäß FIG 6 unterscheidet sich von der in den FIG 3 und 4 gezeigten Benutzeroberfläche 37 im Wesentlichen dadurch, dass mehrere Instanzen der GUI-Seiten 36 und/oder 52 der Benutzeroberfläche 37 nebeneinander dargestellt werden und somit gleichzeitig sichtbar sind.

In der Darstellung gemäß FIG 5 weist die Benutzeroberfläche 37 weiterhin zusätzliche Navigations-Schaltflächen 50 auf, durch deren Betätigung wiederum zwischen den angezeigten GUI-Seiten 36 und/oder 52 geblättert werden kann.

Außerdem weist die Benutzeroberfläche 37 zusätzlich eine Menüzeile 80 auf, durch deren Betätigung zwischen den verschiedenen Applikationen 31-34 gewechselt werden kann. Die Menüzeile 80 ist dabei insbesondere Bestandteil eines Rahmens 81 (oder Containers), in dem die bestehenden Instanzen der Applikationen 31-34 ablaufen.

In FIG 6 ist schematisch ein Modell der Komponenten-Architektur einer der Applikationen 31-33 dargestellt. Der Darstellung ist zu entnehmen, dass die Applikation 31-33 aus Komponenten gebildet ist, die in fünf Schichten angeordnet sind.

Die hierarchisch höchste Schicht dieser Architektur ist als Rahmenschicht 90 bezeichnet. Diese Rahmenschicht 90 ist im Beispiel gemäß FIG 6 durch eine Rahmenkomponente 91 gebildet, die den Rahmen 51 bzw. 81 implementiert. Wie aus den FIG 3 bis 5 deutlich wird, wird durch den Rahmen 51 bzw. 81 (und somit durch die Rahmenkomponente 91) festgelegt, wie viele GUI-Seiten 36 oder 52 gleichzeitig angezeigt werden, wie diese GUI-Seiten 36, 52 auf der Bildschirmfläche angeordnet sind und wie die Kontrollübergabe zwischen den nebenläufig existierenden Instanzen der GUI-Seiten 36 und 52 erfolgt, wie diese Instanzen also für eine Nutzerinteraktion aktiviert und deaktiviert sowie auf dem Bildschirm ein- und ausgeblendet werden.

Die Rahmenkomponente 91 wird spezifisch in Abhängigkeit der Art des Device 8, auf dem die Applikation 31-33 laufen soll, ausgewählt und ist auf den Formfaktor des jeweiligen Device 8 (also die Größe und Geometrie des Bildschirms) sowie auf die in dem Device 8 vorhandenen Mittel zur Nutzerinteraktion zugeschnitten. Beispielsweise unterstützt eine zum Ablauf auf dem Tablet-Computer 11 oder Smartphone 12 vorgesehene Version der Rahmenkomponente 91 Nutzerinteraktions-Methoden, wie sie für die Nutzerinteraktion über einen Touch-Screen üblich sind (z.B. Verschieben der GUI-Seiten 36 und 52 durch manuelles Wischen über den Bildschirm, Zoomen der Bildinformation durch Zwei-Finger-Operationen, Drehen der Bildinformation bei Verkippung des Devices 8 etc.). Eine für den Ablauf in einem Personal-Computer 9 ausgebildete Version der Rahmenkomponente 91 stellt dagegen bevorzugt in Anpassung an eine Nutzerinteraktion mittels Maus oder Touch-Pad grafische Steuerelemente in Form von Schaltflächen (Buttons) zur Verfügung.

Der Rahmenschicht 90 unterlagert ist eine View-Schicht 92. Diese View-Schicht 92 umfasst im Falle der Applikation 31-33 zwei Komponenten 93 und 94, die den "View", das heißt die grafische Gestaltung der GUI-Seiten 37 definieren. Die Komponente 93 definiert hierbei die Steuerelemente der GUI-Seite 36 hinsichtlich Art, Aussehen und Anordnung. Die Komponente 94 definiert entsprechend die Steuerelemente der GUI-Seite 52.

Die Komponenten 93 und 94 sind spezifisch auf die Art der anzuzeigenden Datensätze ausgelegt, im Falle der Applikation 31 also auf Patientendatensätze P. Die Komponenten 93 und 94 der Applikation 31 sind insofern verschieden von entsprechenden Komponenten 93 und 94 der Applikationen 32 und 33 ausgebildet. Sofern die Applikation - abweichend von FIG 6 - zur Anzeige mehrerer Datenarten ausgebildet ist, umfasst die Applikation in der View-Schicht 92 für jede anzuzeigende Datenart jeweils zwei Komponenten, von denen eine die GUI-Seite 36 zur Anzeige der zugehörigen Feeds 35 definiert, während die andere GUI-Seite 52 zur Anzeige der zugehörigen Tweets 27 definiert.

Unterhalb der View-Schicht 92 umfasst die Applikation 31-33 in einer ViewModel-Schicht 95 zwei weitere Komponenten 96 und 97. Jede dieser Komponenten 96 und 97 definiert für die jeweils zugeordnete Komponente 93 bzw. 94 der View-Schicht 92 Eigenschaften und Befehle für die in dieser Komponente 93 bzw. 94 definierten Steuerelemente der graphischen Benutzeroberfläche 37, insbesondere die vorstehend beschriebenen CRUD-Funktionen. Die Komponenten 96 und 97 der ViewModel-Schicht 95 sind hierbei generisch, also unabhängig von der anzuzeigenden Datenart gestaltet. Somit sind die Komponenten 96 und 97 auch bei allen Applikationen 31,32 und 33 identisch.

Unterhalb der ViewModel-Schicht 95 umfasst die Applikation 31-33 eine Model-Schicht 98. Diese Model-Schicht 98 umfasst eine Komponente 99, die die Struktur der anzuzeigenden Datensätze - im Falle der Applikation 31 also die Struktur der Patientendatensätze P - definiert. Die Komponente 99 enthält hierbei insbesondere die Variablen-Definitionen für die Variablen der jeweiligen Datensätze, im Falle der Patientendatensätze P zum Beispiel "Name", "Vorname", etc.

Wiederum unterhalb der Model-Schicht 98 ist eine Treiberschicht 100 angeordnet. Die Treiberschicht 100 umfasst hierbei einen oder mehrere Treiberkomponenten 101, die für die übergeordneten Schichten den Zugriff auf die in einem vorgegebenen Datenspeicher 102 hinterlegten Datensätze und die zugeordneten Feeds 35 und Tweets 27 vermitteln.

Bei dem Datenspeicher 102 handelt es sich, je nach Ausbildung der Treiberkomponente 101, entweder um den lokalen Speicher es Devices 8, den Table-Storage 26 oder einen anderen Speicher des zugeordneten Hubs 22-24 oder einen sonstigen externen Speicher. Im verallgemeinerten Sinne kann es sich bei dem Datenspeicher 102 auch um einen E-Mail- oder SMS-Empfänger, oder ein soziales Netzwerk, insbesondere Twitter, handeln. Die Applikation 31-33 umfasst hierbei vorzugsweise mehrere verschiedenartige Treiberkomponenten 101, die jeweils einem bestimmten Datenspeicher 102 zugeordnet sind.

Alle Treiberkomponenten 101 beruhen auf einer gemeinsamen, festgelegten Treiber-API 103. Die Treiberkomponenten 101 sind ebenfalls generisch, also unabhängig von der anzuzeigenden Datenart.

Die Kommunikation zwischen der oder jeder Treiberkomponente 101 und den übergeordneten Schichten erfolgt hierbei asynchron. Das bedeutet, dass die Komponenten der übergeordneten Schichten nach einer Anfrage an die oder eine der Treiberkomponenten 101 nicht auf die Antwort der Treiberkomponente 101 warten, so dass etwaige Verzögerungen beim Zugriff auf die Datensätze den Ablauf der Applikation 31 nicht belasten.

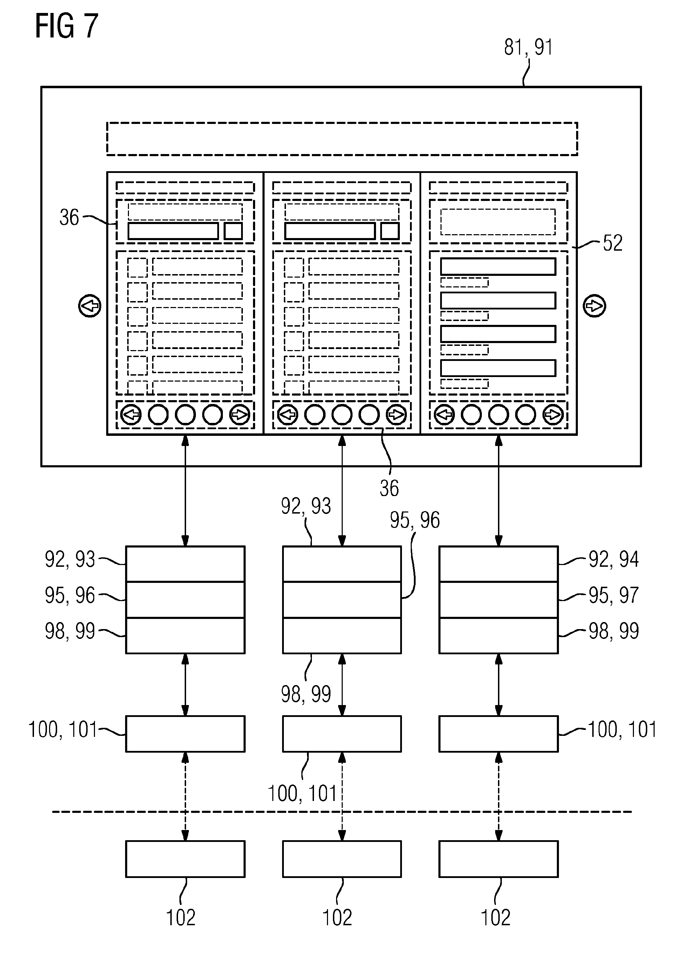

Wie vorstehend beschrieben, können mehrere Instanzen der GUI-Seiten 36 und 52 nebenläufig betrieben werden. Entsprechend können - wie in FIG 7 dargestellt - auch die Komponenten 93,94,96,97,99 der View-Schicht 92, der Viewmodel-Schicht 95 und der Model-Schicht 98 mehrfach instanziiert werden und nebenläufig ablaufen. Auch sind die Treiberkomponenten 101 der Treiberschicht 100 mehrfach instanziierbar, so dass jeder Instanz der GUI-Seite 36 bzw. 52 eine zugehörige Instanz der Treiberkomponente 101 zugeordnet ist. Die Treiberinstanzen werden nebenläufig betrieben und können - wie vorstehend erwähnt - auf denselben Datenspeicher 102 oder auf unterschiedliche Datenspeicher 102 zugreifen.

Die Erfindung wird an den vorstehend beschriebenen Ausführungsbeispielen besonders deutlich, ist gleichwohl auf diese aber nicht beschränkt. Vielmehr können weitere Ausführungsformen der Erfindung aus der vorliegenden Beschreibung und den Ansprüchen abgeleitet werden. Insbesondere können die anhand der Ausführungsbeispiele beschriebenen Einzelmerkmale der Erfindung auch in anderer Weise kombiniert werden, ohne von der Erfindung abzuweichen.

In den nachfolgenden Anhängen sind Beispiele für eine konkrete Implementierung des Applikations-Template, der Treiber-API 103 und der Komponente 97 der ViewModel-Schicht 95 aufgeführt.

### Anhang 1: Beispielhafte Implementierung des Applikations-Template

```
 //-----------------------------------------------------------
 ---------------
 // AppConfig.xml
 //-----------------------------------------------------------
 ---------------
 <?xml version="1.0" encoding="utf-8" ?>
 <Apps>
 <!---------------------------------------------------------
 ----------------->
 <!-- Application 1 -->
 <!---------------------------------------------------------
 ----------------->
 <App>
  <A_AppTitle>patient hub SINGAPORE@W8 kd-24-12-
  2011</A_AppTitle>
  <A_PanoramaBackgroundImaUri>http://singapore4.blob.core.windo
  ws.net/
  mysingapore/thumbs/syngo.via_1.jpg</A-PanoramaBackgroundImaUr
  i>
  <A_NormalMainPage>NormalMainPage1.xaml</A_NormalMainPage>
  <A_PivotMainPage>PivotMainPage1.xaml</A_PivotMainPage>
  <A_PanoramaMainPage>PanoramaMainPage1</A_PanoramaMainPage>
  <A_MainPage>MainPage1.xaml</A_MainPage>
  <AppFeeds>
    <!-- AppFeed 1 -->
    <AppFeed>
      <A_Name>Patient</A_Name>
      <V_Name>PatientView1</V_Name>
      <V_UserControlGuiName>all</V_UserControlGuiName>
      <V_SearchString></V_SearchString>
      <V_NextAppNavUri>Application 2</V_NextAppNavUri>
      <V_PushNotificationChanName>App1-Patient-Notif Chan-
      nel
                         </V_PushNotificationChanName>
      <V_ListView>PatientView.xaml</V_ListView>
      <V_ListItemView>PatientItemView.xaml</V_ListItemView>
      <M_Model>Patient</M_Model>
      <D_Account>singapore4</D_Account>
      <D_Key>s0Zh73EV6WqkQp9CjATic7vEYC4k+ .... </D_Key>
      <D_Table>patients</D_Table>
      <D_Type>AzureTableDriver</D_Type>
    </AppFeed>
    <!-- AppFeed 2 -->
    <AppFeed>
      <A_Name>Patient</A_Name>
      <V_Name>PatientView2</V_Name>
      <V_UserControlGuiName>scheduled</V_UserControlGuiName>
      <V_SearchString></V_SearchString>
      <V_NextAppNavUri>Application 2</V_NextAppNavUri>
      <V_PushNotificationChanName>App1-Patient-Notif Chan-
      nel
                         </V_PushNotificationChanName>
      <V_ListView>PatientView.xaml</V_ListView>
      <V_ListItemView>PatientItemView.xaml</V_ListItemView>
      <M_Model>Patient</M_Model>
      <D_Account>singapore4</D_Account>
      <D_Key>s0Zh73EV6WqkQp9CjATic7vEYC4k+ .... </D_Key>
      <D_Table>patients</D_Table>
      <D_Type>AzureTableDriver</D_Type>
    </AppFeed>
    <!-- AppFeed 3 -->
    <AppFeed>
      <A_Name>Patient</A_Name>
      <V_Name>PatientView3</V_Name>
      <V_UserControlGuiName>billed</V_UserControlGuiName>
      <V_SearchString></V_SearchString>
      <V_NextAppNavUri>Application 2</V_NextAppNavUri>
      <V_PushNotificationChanName>App1-Patient-Notif Chan-
      nel
                         </V_PushNotificationChanName>
      <V_ListView>PatientView.xaml</V_ListView>
      <V_ListItemView>PatientItemView.xaml</V_ListItemView>
      <M_Model>Patient</M_Model>
      <D_Account>singapore4</D_Account>
      <D_Key>s0Zh73EV6WqkQp9CjATic7vEYC4k+ .... </D_Key>
      <D_Table>patients</D_Table>
      <D_Type>AzureTableDriver</D_Type>
    </AppFeed>
  </AppFeeds>
  </App>
  <!---------------------------------------------------------
  ----------------->
  <!-- Application 2 -->
  <!---------------------------------------------------------
  ----------------->
  <App>
  <A_AppTitle>worklist hub GLOBAL SHARED WORKLIST
  </A_AppTitle>
  <A_PanoramaBackgroundImaUri>http://singapore4.blob.core.windo
  ws.net/
  mysingapore/thumbs/syngo.via_1.jpg</A-PanoramaBackgroundImaUr
  i>
  <A_NormalMainPage>NormalMainPage2.xaml</A_NormalMainPage>
  <A_PivotMainPage>PivotMainPage2.xaml</A_PivotMainPage>
  <A_PanoramaMainPage>PanoramaMainPage2</A_PanoramaMainPage>
  <A_MainPage>MainPage2.xaml</A_MainPage>
  <AppFeeds>
    <!-- AppFeed 2 -->
    <AppFeed>
      <A_Name>Worklist</A_Name>
      <V_Name>WorklistView1</V_Name>
      <V_UserControlGuiName>all</V_UserControlGuiName>
      <V_SearchString></V_SearchString>
      <V_NextAppNavUri>Application 3</V_NextAppNavUri>
      <V_PushNotificationChanName>App2-Patient-Notif Chan-
      nel
                         </V_PushNotificationChanName>
      <V_ListView>WorklistView.xaml</V_ListView>
      <V_ListItemView>WorklistItemView.xaml</V_ListItemView>
      <M_Model>Worklist</M_Model>
      <D_Account>singapore4</D_Account>
      <D_Key>s0Zh73EV6WqkQp9CjATic7vEYC4k+ .... </D_Key>
      <D_Table>workitems</D_Table>
      <D_Type>AzureTableDriver</D_Type>
    </AppFeed>
    <!-- AppFeed 2 -->
    <AppFeed>
      <A_Name>Worklist</A_Name>
      <V_Name>WorklistView2</V_Name>
      <V_UserControlGuiName>unread</V_UserControlGuiName>
      <V_SearchString></V_SearchString>
      <V_NextAppNavUri>Application 3</V_NextAppNavUri>
      <V_PushNotificationChanName>App2-Patient-Notif Chan-
      nel
                         </V_PushNotificationChanName>
      <V_ListView>WorklistView.xaml</V_ListView>
      <V_ListItemView>WorklistItemView.xaml</V_ListItemView>
      <M_Model>Worklist</M_Model>
      <D_Account>singapore4</D_Account>
      <D_Key>s0Zh73EV6WqkQp9CjATic7vEYC4k+ .... </D_Key>
      <D_Table>workitems</D_Table>
      <D_Type>AzureTableDriver</D_Type>
    </AppFeed>
    <!-- AppFeed 3 -->
    <AppFeed>
      <A_Name>Worklist</A_Name>
      <V_Name>WorklistView3</V_Name>
      <V_UserControlGuiName>read</V_UserControlGuiName>
      <V_SearchString></V_SearchString>
      <V_NextAppNavUri>Application 3</V_NextAppNavUri>
      <V_PushNotificationChanName>App2-Patient-Notif Chan-
      nel
                         </V_PushNotificationChanName>
      <V_ListView>WorklistView.xaml</V_ListView>
      <V_ListItemView>WorklistItemView.xaml</V_ListItemView>
      <M_Model>Worklist</M_Model>
      <D_Account>singapore4</D_Account>
      <D_Key>s0Zh73EV6WqkQp9CjATic7vEYC4k+ .... </D_Key>
      <D_Table>workitems</D_Table>
      <D_Type>AzureTableDriver</D_Type>
    </AppFeed>
  </AppFeeds>
  </App>
  <!---------------------------------------------------------
  ----------------->
  <!-- Application 3 -->
  <!---------------------------------------------------------
  ----------------->
  <App>
  <A_AppTitle>image hub CONTEXT
  FOLDER </A_AppTitle>
  <A_PanoramaBackgroundImaUri>http://singapore4.blob.core.windo
  ws.net/
  mysingapore/thumbs/syngo.via_1.jpg</A-PanoramaBackgroundImaUr
  i>
  <A_NormalMainPage>NormalMainPage3.xaml</A_NormalMainPage>
  <A_PivotMainPage>PivotMainPage3.xaml</A_PivotMainPage>
  <A_PanoramaMainPage>PanoramaMainPage3</A_PanoramaMainPage>
  <A_MainPage>MainPage3.xaml</A_MainPage>
  <AppFeeds>
    <!-- AppFeed 1 -->
    <AppFeed>
      <A_Name>Context</A_Name>
      <V_Name>ContextView1</V_Name>
      <V_UserControlGuiName>head</V_UserControlGuiName>
      <V_SearchString>ARegion eq 'Head'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 2 -->
    <AppFeed>
      <A_Name>Context</A_Name>
      <V_Name>ContextView2</V_Name>
      <V_UserControlGuiName>thorax</V_UserControlGuiName>
      <V_SearchString>ARegion eq 'Thorax'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 3 -->
    <AppFeed>
      <A_Name>Context</A_Name>
      <V_Name>ContextView3</V_Name>
      <V_UserControlGuiName>body</V_UserControlGuiName>
      <V_SearchString>ARegion eq
      'WholeBody'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 4 -->
    <AppFeed>
      <A_Name>Context</A_Name>
      <V_Name>ContextView4</V_Name>
      <V_UserControlGuiName>key ima</V_UserControlGuiName>
      <V_SearchString>ARegion eq
      'KeyImage'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 5 -->
    <AppFeed>
      <A_Name>Context</A_Name>
      <V_Name>ContextView5</V_Name>
      <V_UserControlGuiName>photo</V_UserControlGuiName>
      <V_SearchString>ARegion eq 'Photo'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 6 -->
    <AppFeed>
      <A_Name>Context</A_Name>
      <V_Name>ContextView6</V_Name>
      <V_UserControlGuiName>video</V_UserControlGuiName>
      <V_SearchString>ARegion eq 'Media'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 7 -->
    <AppFeed>
      <A_Name>Context</A_Name>
      <V_Name>ContextView7</V_Name>
      <V_UserControlGuiName>documents</V_UserControlGuiName>
      <V_SearchString>ARegion eq
      'Document'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 8 -->
    <AppFeed>
      <A_Name>Context</A_Name>
      <V_Name>ContextView8</V_Name>
      <V_UserControlGuiName>all</V_UserControlGuiName>
      <V_SearchString></V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>ContextView.xaml</V_ListView>
      <V_ListItemView>ContextItemView.xaml</V_ListItemView>
      <M_Model>Context</M_Model>
      <D_Account>singapore4</D_Account>
      <D_Key>s0Zh73EV6WqkQp9CjATic7vEYC4k+ .... </D_Key>
      <D_Table>contextfolder</D_Table>
      <D_Type>AzureTableDriver</D_Type>
    </AppFeed>
    <!-- AppFeed 9 -->
    <AppFeed>
      <A_Name>Context</A_Name>
      <V_Name>ContextView9</V_Name>
      <V_UserControlGuiName>files</V_UserControlGuiName>
      <V_SearchString>File eq "</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>FileView.xaml</V_ListView>
      <V_ListItemView>FileItemView.xaml</V_ListItemView>
      <M_Model>File</M_Model>
      <D_Account>singapore4</D_Account>
      <D_Key>s0Zh73EV6WqkQp9CjATic7vEYC4k+ .... </D_Key>
      <D_Table></D_Table>
      <D_Type>IsoDeviceDriver</D_Type>
    </AppFeed>
    <!-- AppFeed 10 -->
    <AppFeed>
      <A_Name>Context</A_Name>
      <V_Name>ContextView10</V_Name>
      <V_UserControlGuiName>blobs</V_UserControlGuiName>
      <V_SearchString>blob eq 'config'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>BlobView.xaml</V_ListView>
      <V_ListItemView>BlobItemView.xaml</V_ListItemView>
      <M_Model>Blob</M_Model>
      <D_Account>singapore4</D_Account>
      <D_Key>s0Zh73EV6WqkQp9CjATic7vEYC4k+ .... </D_Key>
      <D_Table>config</D_Table>
      <D_Type>AzureBlobDriver</D_Type>
    </AppFeed>
    <!-- AppFeed 11 -->
    <AppFeed>
      <A_Name>Context</A_Name>
      <V_Name>ContextView11</V_Name>
      <V_UserControlGuiName>queues</V_UserControlGuiName>
      <V_SearchString>Queue eq
      'modworklist'</V_SearchString>
      <V_NextAppNavUri>Application 4</V_NextAppNavUri>
      <V_PushNotificationChanName>App3-Patient-Notif Chan-
      nel
                         </V_PushNotificationChanName>
      <V_ListView>QueueView.xaml</V_ListView>
      <V_ListItemView>QueueItemView.xaml</V_ListItemView>
      <M_Model>QueueMessage</M_Model>
      <D_Account>singapore4</D_Account>
      <D_Key>s0Zh73EV6WqkQp9CjATic7vEYC4k+ .... </D_Key>
      <D_Table>modworklist</D_Table>
      <D_Type>AzureQueueDriver</D_Type>
    </AppFeed>
  </AppFeeds>
  </App>
  </Apps>
```

### Anhang 2: Beispielhafte Implementierung der Treiber-API 103 aus FIG 6Treiber-API //----------------

```
 // R-MVVM-D - Model and the Driver-API
 //-----------------------------------------------------------
 ---------------
  public class DriverBase<T> : DependencyObject,
  INotifyPropertyChanged
  {
      public event PropertyChangedEventHandler
      PropertyChanged;
      protected void OnNotifyPropertyChanged(string p)
      {
          if (PropertyChanged != null)
          {
              PropertyChanged(this, new
              PropertyChangedEventArgs(p));
          }
      }
      public delegate void
      DriverNotificationEventHandler(object sender, EvtArg e);
      public virtual string Name { get; set; }
      public virtual string Account { get; set; }
      public virtual string Key { get; set; }
      public virtual string Table { get; set; }
      public virtual IStorageCredentials Creds { get; set;
      }
      public virtual CloudTableClient Client { get; set; }
      public virtual TableServiceContext Ctx { get; set; }
      public virtual T SelectedItem { get; set; }
      public virtual int SelectedIndex { get; set; }
      public virtual string State { get; set; }
      public virtual string SearchText { get; set; }
      public virtual string SelectText { get; set; }
      public virtual string BindingItemSource { get; set; }
```

public virtual string ItemType { get; set; }//"Object","File","Blob","Msg" public virtual int AppIndex { get; set; } //normal/pivot/panorama app index public virtual DataServiceCollection<T> ListItems { get; set; } public virtual ObservableCollection<ListItemViewModel<T» ListItemsNonOdata { get; set; } public virtual void GeneralModelNotifier(string reason, string param) { } public virtual void init(DriverNotificationEventHandler han) { } public virtual void ex-it(DriverNotificationEventHandler han) { } public virtual void initTable() { } public virtual void exitTable() { } public virtual void BeginCreateTable() { } public virtual void BeginDeleteTable() { } public virtual void BeginReadTable() { } public virtual void BeginReadEntity() { } public virtual void BeginAddEntity(T list) { } public virtual void BeginUpdateEntity(T list, T oldList) { } public virtual void BeginRemoveEntity(T list) { } }

### Anhang 3: Generisches ListItemViewModel (Beispielhafte Implementierung der Komponente 97 der ViewModelschicht 95 aus FIG 6)

```
 #define GoBackModeInsteadOfNavigate
 using System;
 using System.Collections.Generic;
 using System.Linq;
 using System.Net;
 using System.Windows;
 using System.Windows.Input;
 using System.Windows.Controls;
 using Microsoft.Samples.WindowsPhoneCloud.StorageClient;
 using Microsoft.Samples.Data.Services.Common;
 using System.Windows.Navigation;
 using Microsoft.Phone.Shell;
 using System.Globalization;
 using System.Windows.Documents;
 using System.Windows.Media;
 using System.Windows.Media.Animation;
 using System.Windows.Shapes;
 using Microsoft.Phone.Controls;
 using System.IO;
 namespace AzureTableStore_CRUD_Sample
 {
  public class ListItemViewModel<T> : ViewModelBase
  {
      private string _bindingItemSource;
      private string _name;
      private Object _model;
      private Object _oldmodel;
      private T _dataModel;
      private string _appTitle;
      private string _account;
      private string _table;
      private int _selectedIndex;
      private string _lastExportedIsoEntityUriString;
      private string
      _lastExportedAzureCloudEntityUriString;
      private string _lastExportedWebEntityUriString;
      UploadFile pUploader;
      public ListItemViewModel()
      {
      }
      #if DataModelInheritanceUsed
      public ListItemViewModel(long p) : base(p)
      {
      }
      public ListItemViewModel(bool set) : base(set)
      {
      }
      public ListItemViewModel(ICloudBlobContainer o)
          : base(o)
      {
      }
      #else
      #endif
      public string BindingItemSource
      {
          get { return bindingItemSource; }
          set { bindingItemSource = value; }
      }
      public string Name
      {
          get { return _name; }
          set { _name = value; }
      }
      public string Account
      {
          get { return _account; }
          set { _account = value; }
      }
      public string Table
      {
          get { return _table; }
          set { _table = value; }
      }
      public int SelectedIndex
      {
          get { return _selectedIndex; }
          set { _selectedIndex = value; }
      }
      public Object Model
      {
          get { return _model; }
          set { _model = value; }
      }
      public Object oldModel
      {
          get { return _oldmodel; }
          set { _oldmodel = value; }
      }
      public T DataModel
      {
          get { return _dataModel; }
          set { _dataModel = value; }
      }
      public string AppTitle
      {
          get { return _appTitle; }
          set { _appTitle = value; }
      }
      public event EventHandler<NavigationEventArgs> Navi-
      gate;
      public void NavigateTo(Uri navigationUri)
      {
          this.RaiseNavigate(navigationUri);
      }
      private void RaiseNavigate(Uri uri)
      {
          var navigate = this.Navigate;
          if (navigate != null)
          {
              navigate(this, new NavigationEventArgs(null,
              uri));
          }
      }
      private void
      ApplicationBarIconButton_CANCEL_INPUT_Click()
      {
          SetApplicationState("CurrentResponse", "Cancel");
          SetApplicationState("CurrentViewModel",
          this.Model);
          #if GoBackModeInsteadOfNavigate1
          (Application.Current.RootVisual as
          PhoneApplicationFrame).GoBack();
          #else
          NavigateBackToCaller();
          #endif
      }
      private void
      ApplicationBarIconButton_SAVE_ENTITY_Click()
      {
          SetApplicationState("CurrentResponse", "Save");
          object focusObj =
          FocusManager.GetFocusedElement();
          if (focusObj != null && focusObj is TextBox)
          {
              var binding = (focusObj as Text-
              Box).GetBindingExpression(TextBox.TextProperty);
              binding.UpdateSource();
          }
          SetApplicationState("CurrentViewModel",
          this.Model);
          SetApplicationState("OldViewModel",
          this.oldModel);
          #if GoBackModeInsteadOfNavigate1
          (Application.Current.RootVisual as
          PhoneApplicationFrame).GoBack();
          #else
          NavigateBackToCaller();
          #endif
      }
      private void
      ApplicationBarIconButton_REMOVE_ENTITY_Click()
      {
          SetApplicationState("CurrentResponse", "Delete");
          SetApplicationState("CurrentViewModel",
          this.Model);
          #if GoBackModeInsteadOfNavigate1
          (Application.Current.RootVisual as
          PhoneApplicationFrame).GoBack();
          #else
          NavigateBackToCaller();
          #endif
      }
      public void
      old_ApplicationBarMenueButton_EXPORT_Click()
      {
          MessageBox.Show("Serializing data model to string
          ...") ;
          string data = "";
          data =
          DataContractSerializerHelpers.SerializeToString(this.Model);
          MessageBox.Show("Serializing done! Data=" + da-
          ta);
          switch (App.DstModel)
          {
              case "IsolatedStorage":
                  string StorageFile = Account + "_" + Ta-
                  ble + "_" + SelectedIndex + ".tweet";
                  MessageBox.Show("Exporting data model to
                  app-dir in iso storage, filename=" + StorageFile + " ...");
                  IsoImportExport.WriteStringToIsoFile(data, StorageFile);
                  break;
              case "FileSystemStorage":
                  break;
              case "AzureBlobStorage":
                  break;
              case "WebUriStorage":
                  break;
              default:
                  break;
          }
          MessageBox.Show("Exporting done!");
      }
      public void
      old_ApplicationBarMenueButton_IMPORT_Click()
      {
          string data = "";
          switch (App.SrcModel)
          {
              case "IsolatedStorage":
                  string StorageFile = Account + "_" + Ta-
                  ble + "_" + SelectedIndex + ".tweet";
                  MessageBox.Show("Importing data model to
                  string from app-dir in iso storage, filename=" + StorageFile
                  + " ...");
                  data =
                  IsoImportExport.ReadStringFromIsoFile(StorageFile);
                  break;
              case "FileSystemStorage":
                  break;
              case "AzureBlobStorage":
                  break;
              case "WebUriStorage":
                  break;
              default:
                  break;
          }
          MessageBox.Show("Importing done!");
          MessageBox.Show("Deserializing data model from
          string ...");
          var tweet =
          DataContractSerializerHelpers.DeserializeFromString<T>(data);
          MessageBox.Show("De-Serializing done! Data=" +
          data);
          MessageBox.Show("Copy data from tweet to
          this.Model as <T> ...");
          this.Model = tweet;
          MessageBox.Show("Copy done, this.Model as <T> has
          been filled!");
          MessageBox.Show("Deserializing done!");
      }
       
      // Import-Tweet from external storage
      public void ApplicationBarMenueButton_IMPORT_Click()
      {
          switch (App.SrcModel)
          {
              case "IsolatedStorage":
                  ImportFromIsoEntityUpdate();
                  break;
              case "AzureBlobStorage":
                  ImportFromAzureCloudEntityUpdate();
                  break;
              case "WebUriStorage":
                  ImportFromWebEntityUpdate();
                  break;
              default:
                  break;
          }
      }
      public void ImportFromIsoEntityUpdate()
      {
          int index = SelectedIndex;
          string StorageType = "IsolatedStorage";
          string StorageHostName = "";
          string StorageContainerName = "";
          string StoragePathName = "";
          string StorageFileName = Account + "_" + Table +
          ".tweet";
          ImportEntityUpdate(index, StorageType,
          StorageHostName, StorageContainerName, StoragePathName,
          StorageFileName);
      }
      public void ImportFromAzureCloudEntityUpdate()
      {
          int index = SelectedIndex;
          string StorageType = "AzureBlobStorage";
          string StorageHostName = Account;
          string StorageContainerName =
          App.SrcContTabQueOrDir;
          string StoragePathName = "";
          string StorageFileName = Account + "_" + Table +
          ".tweet";
          ImportEntityUpdate(index, StorageType,
          StorageHostName, StorageContainerName, StoragePathName,
          StorageFileName);
      }
      public void ImportFromWebEntityUpdate()
      {
          int index = SelectedIndex;
          string StorageType = "WebUriStorage";
          string StorageHostName = App.SrcAccountOrHost;
          string StorageContainerName =
          App.SrcContTabQueOrDir;
          string StoragePathName = "";
          string StorageFileName = Account + "_" + Table +
          ".tweet";
          ImportEntityUpdate(index, StorageType,
          StorageHostName, StorageContainerName, StoragePathName,
          StorageFileName);
      }
      public void ImportEntityUpdate(int index, string
      StorageType, string StorageHostName, string
      StorageContainerName, string StoragePathName, string
      StorageFileName)
      {
          string data = "";
          string fnam = StorageHostName +
          StorageContainerName + StoragePathName + StorageFileName;
          switch (StorageType)
          {
              case "IsolatedStorage":
                  data =
                  IsoImportExport.ReadStringFromIsoFile(fnam);
                  break;
              case "FileSystemStorage":
                  MessageBox.Show("FileSystemStorage API is
                  not implemented, maybe you should use IsolatedStorage any-
                  way!");
                  break;
              case "AzureBlobStorage":
                  string url = "http://" + Account +
                  ".blob.core.windows.net" + "/" + StorageContainerName + "/" +
                  StoragePathName + StorageFileName;
                  WebClient client = new WebClient();
                  client.DownloadStringCompleted += new
                  DownloadStringCompletedEventHandler(client_EntityUpdateDownlo
                  adStringCompleted);
                  client.DownloadStringAsync(new Uri(url,
                  UriKind.Absolute), fnam);
                  return;
              case "WebUriStorage":
                  string url1 = "http://" + StorageHostName
                  + "/" + StorageContainerName + "/" + StorageFileName;
                  WebClient client1 = new WebClient();
                  client1.DownloadStringCompleted += new
                  DownloadStringCompletedEventHandler(client_EntityUpdateDownlo
                  adStringCompleted);
                  client1.DownloadStringAsync(new Uri(url1,
                  UriKind.Absolute), fnam);
                  return;
              default:
                  break;
          }
          var tweet =
          DataContractSerializerHelpers.DeserializeFromString<T>(data);
          this.Model = tweet;
      }
      void cli-
      ent_EntityUpdateDownloadStringCompleted(object sender,
      DownloadStringCompletedEventArgs e)
      {
          if (e.Error == null)
          {
              var tweet =
              DataContractSerializerHelpers.DeserializeFromString<T>(e.Resu
              lt);
              this.Model = tweet;
              IsoImportExport.WriteStringToIsoFile(tweet as
              string, e.UserState as string);
          }
          else
          {
              MessageBox.Show(e.Error.ToString());
          }
      }
       
      // Export-Tweet to external storage
        
      public void ApplicationBarMenueButton_EXPORT_Click()
      {
          switch (App.DstModel)
          {
              case "IsolatedStorage":
                  _lastExportedIsoEntityUriString =
                  ExportEntityToIso();
                  break;
              case "AzureBlobStorage":
                  _lastExportedAzureCloudEntityUriString =
                  ExportEntityToAzureCloud();
                  break;
              case "WebUriStorage":
                  _lastExportedWebEntityUriString =
                  ExportEntityToWeb();
                  break;
              default:
                  break;
          }
      }
      public string ExportEntityToIso()
      {
          int index = SelectedIndex;
          string StorageType = "IsolatedStorage";
          string StorageHostName = "";
          string StorageContainerName = "";
          string StoragePathName = "";
          string StorageFileName = Account + "_" + Table +
          ".tweet";
          return ExportEntity(index, StorageType,
          StorageHostName, StorageContainerName, StoragePathName,
          StorageEileName);
      }
      public string ExportEntityToAzureCloud()
      {
          int index = SelectedIndex;
          string StorageType = "AzureBlobStorage";
          string StorageHostName = "";
          string StorageContainerName =
          App.DstContTabQueOrDir;
          string StoragePathName = "";
          string StorageFileName = Account + "_" + Table +
          ".tweet";
          return ExportEntity(index, StorageType,
          StorageHostName, StorageContainerName, StoragePathName,
          StorageFileName);
      }
      public string ExportEntityToWeb()
      {
          int index = SelectedIndex;
          string StorageType = "WebUriStorage";
          string StorageHostName = App.SrcAccountOrHost;
          string StorageContainerName =
          App.DstContTabQueOrDir;
          string StoragePathName = "";
          string StorageFileName = Account + "_" + Table +
          ".tweet";
          return ExportEntity(index, StorageType,
          StorageHostName, StorageContainerName, StoragePathName,
          StorageFileName);
      }
      public string ExportEntity(int index, string
      StorageType, string StorageHostName, string
      StorageContainerName, string StoragePathName, string
      StorageFileName)
      {
          string uriAsString = "";
          string data = "";
          data =
          DataContractSerializerHelpers.SerializeToString(this.Model);
          switch (StorageType)
          {
              case "IsolatedStorage":
              IsoImportExport.WriteStringToIsoFile(data, StorageFileName);
                  uriAsString = StorageFileName;
                  break;
              case "FileSystemStorage":
                  MessageBox.Show("FileSystemStorage API is
                  not implemented, maybe you should use IsolatedStorage any-
                  way!");
                  uriAsString = "";
                  break;
              case "AzureBlobStorage":
              IsoImportExport.WriteStringToIsoFile(data, StorageFileName);
                  byte[] b = Con-
                  vert.FromBase64String(data);
                  Stream memStream = new MemoryStream(b);
                  if (pUploader == null) pUploader = new
                  UploadFile();
                  string ret =
                  pUploader.DoFileUpload(memStream, StoragePathName,
                  StorageFileName, App.File_TimeOutSeconds,
                  App.File_SasExpired, App.File_SasUrl);
                  if (ret == "Blocked")
                  {
                      MessageBox.Show("Upload is currently
                      blocked, try again!");
                      uriAsString = "";
                      return uriAsString;
                  }
                  uriAsString = "http://" + Account +
                  ".blob.core.windows.net" + "/" + StorageContainerName + "/" +
                  StoragePathName + StorageFileName;
                  break;
              case "WebUriStorage":
                  string url = "http://" + StorageHostName
                  + "/" + StorageContainerName + "/" + StorageFileName;
                  Uri uri = new Uri(url, UriKind.Absolute);
                  WebImportExport.WriteStringToWebFile(uri,
                  data);
                  uriAsString = url;
                  break;
              default:
                  uriAsString = "";
                  break;
          }
          return uriAsString;
      }
      public void
      ItemViewPopulateApplicationBarButtons (IApplicationBar
      applicationBar)
      {
          applicationBar.Buttons.Clear();
          applicationBar.MenuItems.Clear();
          var saveButton = new ApplicationBarIconButton(new
          Uri(string.Format(CultureInfo.InvariantCulture, "{0}{1}",
              "/Toolkit.Content/",
              "ApplicationBar.Save.png"), UriKind.Relative)) { Text =
              "SAVE" };
          saveButton.Click += (s, e) =>
          this.ApplicationBarIconButton_SAVE_ENTITY_Click();
          applicationBar.Buttons.Add(saveButton);
          var cancelButton = new
          ApplicationBarIconButton(new
          Uri(string.Format(CultureInfo.InvariantCulture, "{0} {1}",
              "/Toolkit.Content/",
              "ApplicationBar.Cancel.png"), UriKind.Relative)) { Text =
              "CANCEL" };
          cancelButton.Click += (s, e) =>
          this.ApplicationBarIconButton_CANCEL_INPUT_Click();
          applicationBar.Buttons.Add(cancelButton);
          var delButton = new ApplicationBarIconButton(new
          Uri(string.Format(CultureInfo.InvariantCulture, "{0}{1}",
              "/Toolkit.Content/",
              "ApplicationBar.Delete.png"), UriKind.Relative)) { Text =
              "DEL ENTITY" };
          delButton.Click += (s, e) =>
          this.ApplicationBarIconButton_REMOVE_ENTITY_Click();
          applicationBar.Buttons.Add(delButton);
          var exportMenue = new
          ApplicationBarMenuItem("export model data to file ...");
          exportMenue.Click += (s, e) =>
          this.ApplicationBarMenueButton_EXPORT_Click();
          applicationBar.MenuItems.Add(exportMenue);
          var importMenue = new
          ApplicationBarMenuItem("import model data from file into this
          entity ...");
          importMenue.Click += (s, e) =>
          this.ApplicationBarMenueButton_IMPORT_Click();
          applicationBar.MenuItems.Add(importMenue);
      }
      private void NavigateBackToCaller()
      {
          int SelectedIndex = -1;
          var destination = (Uri)null;
          if (App.DesignerMode.ToUpper() == "NORMAL" | |
          App.DesignerMode == null | | App.DesignerMode.ToUpper() == "")
              destination = new
              Uri("/Views/NormalMainPage.xaml?selectedItem=" +
              SelectedIndex, UriKind.Relative);
          if (App.DesignerMode.ToUpper() == "PIVOT")
              destination = new
              Uri("/Views/PivotMainPage.xaml?selectedItem=" +
              SelectedIndex, UriKind.Relative);
          if (App.DesignerMode.ToUpper() == "PANORAMA")
              destination = new
              Uri("/Views/PanoramaMainPage.xaml?selectedItem=" +
              SelectedIndex, UriKind.Relative);
          this.RaiseNavigate(destination);
      }
      private void SetApplicationState(string key, object
      value)
      {
          if
          (PhoneApplicationService.Current.State.ContainsKey(key))
          {
          PhoneApplicationService.Current.State.Remove(key);
          }
          PhoneApplicationService.Current.State.Add(key,
          value);
      }
      private F GetApplicationState<F>(string key)
      {
          if
          (!PhoneApplicationService.Current.State.ContainsKey(key))
          {
              return default(F);
          }
          return
          (F)PhoneApplicationService.Current.State[key];
      }
      private void RemoveApplicationState(string key)
      {
          if
          (PhoneApplicationService.Current.State.ContainsKey(key))
          {
          PhoneApplicationService.Current.State.Remove(key);
          {
      }
  }
  }
```

### Bezugszeichenliste

- 1: System
- 2,2': (medizinische) Einrichtung
- 3: Modalität
- 4: Computertomograph
- 5: C-Bogengerät
- 6: Magnetresonanztomograph
- 7: (Steuer- und Auswerte-)Rechner
- 8: Device
- 9: Personal-Computer
- 10: Bildschirm
- 11: Tablet-Computer
- 12: Smartphone
- 13: Public Cloud
- 14: (Datenübertragungs-)Netz
- 15: Intranet
- 16: Internet
- 17: Firewall
- 18,18': Subscription
- 19: Cloud Storage
- 20: App Store
- 21: Cloud-Compute-Service
- 22: (Patient-)Hub
- 23: (Worklist-)Hub
- 24: (Context-)Hub
- 25: (Image-)Hub
- 26: Table-Storage
- 27: Tweet
- 28: Blob-Storage
- 29: URI
- 31: Applikation
- 32: Applikation
- 33: Applikation
- 34: Applikation
- 35: Feed
- 36: GUI-Seite
- 37: (graphische) Benutzeroberfläche
- 40: Kopfzeile
- 41: Suchfeld
- 42: Anzeigefeld
- 43: Befehlsleiste
- 44: Eingabezeile
- 45: Schaltfläche
- 46: Vorauswahlfeld
- 47: Bild
- 48: Schlagwort
- 49: Schaltfläche
- 50: Schaltfläche
- 51: Rahmen
- 52: GUI-Seite
- 60: Kopfzeile
- 61: Überschrift
- 62: Anzeigefeld
- 63: Befehlszeile
- 64: Schaltfläche
- 65: Schaltfläche
- 67: Feldnamen
- 68: Feldinhalt
- 69: Seite
- 70: Kopfzeile
- 71: Überschrift
- 72: Anzeigefeld
- 73: Befehlszeile
- 74: Schaltfläche
- 80: Menüzeile
- 81: Rahmen
- 90: Rahmenschicht
- 91: Rahmenkomponente
- 92: View-Schicht
- 93: Komponente
- 94: Komponente
- 95: ViewModel-Schicht
- 96: Komponente
- 97: Komponente
- 98: Model-Schicht
- 99: Komponente
- 100: Treiberschicht
- 101: Treiberkomponente
- 102: Datenspeicher
- 103: Treiber-API

- P: Patientendatensatz
- W: Aufgabe
- C: Kontextdatensatz
- B: Bilddatensatz
- V: Ansicht
- Q: Suchanfrage
- R: Anfrage
- K: Keyimage

## Patentansprüche

1. System (1) zur Verwaltung und Bearbeitung von Daten einer medizinischen Einrichtung (2,2'), mit mindestens einer Browser-Applikation (31-33) zum Ablauf auf einem Nutzergerät (8), wobei die Browser-Applikation (31-33) dazu eingerichtet ist,
- Datensatzlisten (35), die zu einer Anzahl von medizinischen Datensätzen (P,W,C) mindestens einer bestimmten Datenart jeweils einen Listeneintrag (27) enthalten, sowie
- einzelne Listeneinträge (27)
zur Ansicht und Bearbeitung anzuzeigen, wobei die Browser-Applikation (31-33) hinsichtlich ihrer Komponenten-Architektur in folgende Schichten gegliedert ist:
- Eine Rahmenschicht (90), umfassend mindestens eine Rahmenkomponente (91), wobei die Rahmenkomponente (91) eine Anzahl und Anordnung von gleichzeitig anzuzeigenden GUI-Seiten (36) für jeweils eine Datensatzliste (35) oder GUI-Seiten (52) für jeweils einen Listeneintrag (27) definiert, und wobei die Rahmenkomponente (91) weiterhin Funktionen für die Kontrollübergabe zwischen verschiedenen GUI-Seiten (36,52) implementiert,
- eine View-Schicht (92), umfassend eine Anzahl von Komponenten (93,94), von denen jede jeweils spezifisch für eine bestimmte Datenart den graphischen Gehalt einer GUI-Seite (36) zur Anzeige einer Datensatzliste (35) oder einer GUI-Seite (52) zur Anzeige eines Listeneintrags (27) definiert,
- eine ViewModel-Schicht (95), umfassend eine Anzahl von Komponenten (96,97), von denen jede für mindestens eine zugeordnete Komponente (93,94) der View-Schicht (92) Eigenschaften und Befehle für die Steuerelemente der dort definierten GUI-Seite (36,52) definiert,
- eine Model-Schicht (98), umfassend eine Anzahl von Komponenten (99), von denen jede jeweils spezifisch für eine bestimmte Datenart die Struktur der zugehörigen Datensätze (P,W,C) definiert, sowie
- eine Treiberschicht (100), umfassend eine Anzahl von Treiberkomponenten (101), von denen jede jeweils den Datenzugriff auf einen bestimmten Datenspeicher (19) innerhalb oder außerhalb des Nutzergeräts (8) vermittelt,
wobei die Komponenten (96,97) der ViewModel-Schicht (95) generisch, das heißt unabhängig von der Datenart sind.

2. System (1) nach Anspruch 1,
wobei die Treiberkomponenten (101) generisch, das heißt unabhängig von der Datenart sind.

3. System (1) nach Anspruch 1 oder 2,
wobei die oder jede Rahmenkomponente (91) generisch, das heißt unabhängig von der Datenart ist.

4. System (1) nach einem der Ansprüche 1 bis 3,
wobei die Treiberschicht (100) eine Anwender-Programmier-Schnittstelle (103) umfasst, auf der die oder jede Treiberkomponente (101) basiert.

5. System (1) nach einem der Ansprüche 1 bis 4,
wobei die Komponenten (96,97) der ViewModel-Schicht (95) generische Funktionen zum Erzeugen, Lesen, Aktualisieren und Löschen von Datensatzlisten (35) oder einzelnen Listeneinträgen (27) umfassen.

6. System (1) nach einem der Ansprüche 1 bis 5,
wobei die Browser-Applikation (31-33) zum Anzeigen von Datensatzlisten (35) und Listeneinträgen (27) zu Datensätzen (P,W,C) mindestens einer der folgenden Datenarten eingerichtet ist:
- Patientendaten, von denen jeder Datensatz (P) jeweils persönliche und medizinische Daten über einen bestimmten Patienten enthält,
- Aufgaben oder Arbeitslisten, von denen jeder Datensatz (W) eine von einem medizinischen Nutzer zu erledigende Aufgabe bzw. Aufgabefolge enthält,
- Kontextdaten, von denen jeder Datensatz (C) Kontextinformation zu einem medizinischen Bilddatensatz (B) insbesondere einen Verweis (29) auf den Speicherort dieses Bilddatensatzes (B) enthält.

7. System (1) nach einem der Ansprüche 1 bis 6,
wobei die Browser-Applikation (31-33) dazu eingerichtet ist, auf Datensätze (P,W,C) und/oder deren zugeordnete Listeneinträge (27) in einem außerhalb des Nutzergeräts (8) angeordneten Datenspeicher (19), insbesondere einem Cloud-Storage (19) einer Public Cloud (13), unmittelbar ohne Inanspruchnahme eines zwischengeschalteten Servers zuzugreifen.

8. System (1) nach einem der Ansprüche 1 bis 7,
mit einem Applikations-Template und einem Applikations-Konfigurator, wobei der Applikations-Konfigurator dazu eingerichtet ist, die oder jede Browser-Applikation (31-33) aus dem Applikations-Template anhand von vorgegebener Konfigurations-Information automatisch zu generieren, wobei die Konfigurations-Information folgende Angaben umfasst:
- Angaben zu dem Nutzergerät (8), auf dem die jeweilige Browser-Applikation (31-33) ablaufen soll,
- Angaben zu der oder jeder Datenart der Datensätze (P,W,C), deren zugeordnete Listeneinträge (27) und Datensatzlisten (35) mittels der jeweiligen Browser-Applikation (31-33) angezeigt und bearbeitet werden sollen, und/oder
- Angaben zu dem oder jeden Datenspeicher (102), auf den die jeweilige Browser-Applikation (31-33) zugreifen soll.

9. System (1) nach einem der Ansprüche 1 bis 8,
wobei die oder jede Treiberkomponente (101) mehrfach instantiierbar ist.

10. System (1) nach einem der Ansprüche 1 bis 9,
wobei der Datenzugriff durch die oder jede Treiberkomponente (101) asynchron und unabhängig von den weiteren Treiberkomponenten (101) vermittelt wird.
